# EUROPÄISCHE PATENTSCHRIFT

(11) **EP 0 495 250 B1**
(45) Veröffentlichungstag und Bekanntmachung des Hinweises auf die Patenterteilung: **31.01.1996**
(21) Anmeldenummer: 91122366.7
(22) Anmeldetag: 30.12.1991
(51) Int. Cl.: C09B 44/10, C07D 285/135, D06P 1/08, C09D 11/00

(54) **Kationische 1,3,4-Thiadiazolfarbstoffe**
Cationic 1,3,4 thiadiazole dyes
Colorants cationiques 1,3,4-thiadiazole

(30) Priorität: 12.01.1991 DE 4100810
(43) Veröffentlichungstag der Anmeldung: 22.07.1992
(73) Patentinhaber: BAYER AG, D-51368 Leverkusen (DE)
(72) Erfinder: Berneth, Horst, Dr., W-5090 Leverkusen 1 (DE); Leverenz, Klaus, Dr., W-5090 Leverkusen 1 (DE)

(56) Entgegenhaltungen:
- EP-A- 0 045 402
- GB-A- 2 017 134

## Beschreibung

In der GB-A 2 017 134 und der EP-A 45 402 sind 1,3,4-Thiadiazol-Azofarbstoffe beschrieben, die in der Kupplungskomponente, soweit sich diese vom Benzol ableitet, frei von zur Azogruppe o-ständigen Acylgruppen sind.

Die vorliegende Erfindung betrifft kationische 1,3,4-Thiadiazolfarbstoffe der Formel (I) 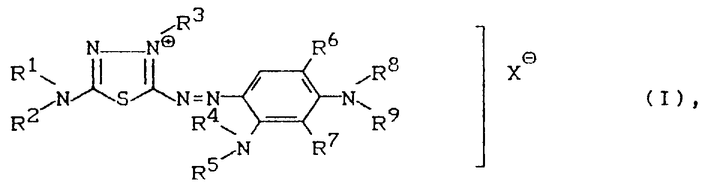 in der
- R¹ und R: unabhängig voneinander Wasserstoff, Alkyl, Alkenyl, Cycloalkyl, Aralkyl, Aryl, einen gegebenenfalls über eine Methylen- oder Ethylenbrücke gebundenen Heterocyclus oder R¹ und R gemeinsam mit dem dazwischenliegenden Stickstoffatom einen Heterocyclus bedeuten,
- R³: Alkyl, Alkenyl oder Aralkyl bedeutet,
- R⁴: Wasserstoff oder Alkyl bedeutet,
- R⁵: eine Acylgruppe bedeutet,
- R⁶ und R⁷: unabhängig voneinander Wasserstoff, Alkyl, Alkoxy, Aryloxy oder Halogen bedeuten und
- R⁸ und R⁹: unabhängig voneinander Wasserstoff, Alkyl, Alkenyl, Cycloalkyl, Aralkyl, Aryl, einen gegebenenfalls über eine Methylen- oder Ethylenbrücke gebundenen Heterocyclus oder R⁸ und R⁹ gemeinsam mit dem dazwischenliegenden Stickstoffatom einen Heterocyclus bedeuten, wobei
- R⁶ und R⁸: und/oder R⁷ und R⁹ gemeinsam auch eine 2- bis 4-gliedrige Brücke bedeuten können, die gegebenenfalls ein Sauerstoff- oder Stickstoffatom enthalten und gegebenenfalls mit 1 bis 3 Alkylgruppen substituiert sein kann und
- X^{⊖}: ein Anion bedeutet und
wobei alle vorhandenen Alkyl-, Alkenyl-, Cycloalkyl-, Aralkyl-, Aryl-, Alkoxy- und heterocyclischen Reste gegebenenfalls mit nichtionogenen Substituenten, Carboxylgruppen, Ammoniumgruppen und/oder Pyridiniumgruppen substituiert sein können, sowie deren Herstellung und Verwendung und die entsprechenden Anhydrobasen.

Bevorzugt sind kationische 1,3,4-Thiadiazolfarbstoffe der Formel (I), bei denen
R⁵ SO₂R¹⁰ oder COR¹¹ bedeutet, wobei
- R¹⁰: für Wasserstoff, Alkyl, Alkenyl, Cycloalkyl, Aralkyl, Aryl oder einen heterocyclischen Rest und
- R¹¹: unabhängig von R¹⁰ die gleichen Bedeutungen wie R¹⁰ hat und zusätzlich auch für O-Alkyl, O-Aryl oder NH-R¹⁰ stehen kann.

Nichtionogene Substituenten sind beispielsweise die in der Farbstoffchemie üblichen, nicht dissoziierenden Substituenten, wie Cyano, Hydroxy, Fluor, Chlor, Brom, Nitro, Alkyl, Monoalkylamino, Dialkylamino, Alkoxy, Phenyl, Acyloxy, Acylamino, Alkoxycarbonyl und Alkoxycarbonyloxy.

Acylreste sind beispielsweise Formyl, Alkylcarbonyl, Alkenylcarbonyl, Arylcarbonyl, Alkoxycarbonyl, Aminocarbonyl, Monoalkylaminocarbonyl, Dialkylaminocarbonyl, Alkylsulfonyl, Arylsulfonyl, Monoalkylaminosulfonyl, Dialkylaminosulfonyl und die Radikale heterocyclischer Carbon- und Sulfonsäuren.

Alkylreste, auch solche in Alkoxy- und Aralkylresten, sind beispielsweise solche mit 1 bis 8, vorzugsweise 1 bis 4 C-Atomen. Sie können auch verzweigt sein.

Alkenylreste sind beispielsweise solche mit 2 bis 6, vorzugsweise 2 bis 3 C-Atomen.

Cycloalkylreste sind beispielsweise solche mit 4 bis 7, vorzugsweise 5 bis 6 C-Atomen.

Halogen steht vorzugsweise für Fluor, Chlor oder Brom.

Arylreste, auch solche in Aralkylresten, sind vorzugsweise Phenylreste, die gegebenenfalls durch 1 bis 3 der oben beschriebenen nichtionogenen Reste und/oder eine Carboxylgruppe substituiert sein können.

Heterocyclische Reste sind beispielsweise Thienyl, Furyl und Pyridyl, sowie ihre teilweise oder vollständig hydrierten Derivate. Sie können gegebenenfalls 1 bis 3 der oben beschriebenen nichtionogenen Reste enthalten.

Als Anionen sind farblose, organische und anorganische Anionen bevorzugt, beispielsweise Fluorid, Chlorid, Bromid, Iodid, Perchlorat, Tetrafluoroborat, Hydroxid, Hydrogensulfat, Sulfat, Dihydrogenphosphat, Hydrogenphosphat, Phosphat, Hydrogencarbonat, Carbonat, Methylsulfat, Ethylsulfat, Cyanat, Thiocyanat, Tri- und Tetrachlorozinkat und Anionen gesättigter oder ungesättigter aliphatischer, cycloaliphatischer, aromatischer oder heterocyclischer Carbon- und Sulfonsäuren wie Formiat, Acetat, Hydroxyacetat, Cyanacetat, Propionat, Hydroxypropionat, Oxalat, Citrat, Lactat, Tartrat, das Anion der Cyclohexancarbonsäure, Phenylacetat, Benzoat, das Anion der Nikotinsäure, Methansulfonat, Ethansulfonat, Benzolsulfonat, Chlorbenzolsulfonat, Toluolsulfonat und Hexafluorosilikat.

Wenn es sich um mehrwertige Anionen handelt, z.B. um Sulfat oder Oxalat, dann steht in Formel (I) X^{⊖} für ein Äquivalent solch eines mehrwertigen Anions.

Bevorzugt sind weiterhin kationische 1,3,4-Thiadiazolfarbstoffe der Formel (I), in der
- R¹ und R: unabhängig voneinander je einen gegebenenfalls durch Hydroxy, Halogen, Cyano, C₁-C₄-Alkoxy, Aminocarbonyl und/oder C₁-C₄-Alkoxycarbonyl substituierten C₁-C₈-Alkylrest, Allyl, Cyclopentyl, Cyclohexyl, einen gegebenenfalls durch Halogen, Cyano, C₁-C₄-Alkyl und/oder C₁-C₄-Alkoxy substituierten Benzyl- oder Phenethylrest, Thienyl-, Furyl-, Tetrahydrofuryl-, Pyridylmethyl- oder Pyridylethylrest, einen gegebenenfalls durch Halogen, Cyano, C₁-C₄-Alkyl und/oder C₁-C₄-Alkoxy substituierten Phenylrest bedeuten, wobei R auch Wasserstoff bedeuten kann, oder
- R¹ und R: gemeinsam mit dem dazwischenliegenden Stickstoffatom einen gegebenenfalls durch bis zu 4 Methylgruppen substituierten Pyrrolidono-, Piperidino-, Piperazino- oder Morpholinorest bedeuten,
- R³: gegebenenfalls durch Hydroxy, Halogen, Cyano, C₁-C₄-Alkoxy, Aminocarbonyl und/oder C₁-C₄-Alkoxycarbonyl substituiertes C₁-C₄-Alkyl, Allyl oder einen gegebenenfalls durch Halogen, C₁-C₄-Alkyl und/oder C₁-C₄-Alkoxy substituierten Benzyl-oder Phenethylrest bedeutet,
- R⁴: Wasserstoff oder C₁-C₄-Alkyl bedeutet,
- R⁵: SO₂R¹⁰ oder COR¹¹ bedeutet,
- R⁶ und R⁷: unabhängig voneinander Wasserstoff, C₁-C₄-Alkyl, C₁-C₄-Alkoxy oder Halogen bedeuten,
- R⁸ und R⁹: unabhängig voneinander einen gegebenenfalls durch Hydroxy, Halogen, Cyano, C₁-C₄-Alkoxy, Aminocarbonyl und/oder C₁-C₄-Alkoxycarbonyl substituierten C₁-C₄-Alkylrest, Allyl, Cyclopentyl, Cyclohexyl, einen gegebenenfalls durch Halogen, C₁-C₄-Alkyl, C₁-C₄-Alkoxy, C₁-C₄-Acylamino und/oder Hydroxy substituierten Benzyl-, Phenethyl- oder Phenylrest, Thienyl-, Furyl-, Tetrahydrofuryl-, Pyridyl-, Pyridylmethyl- oder Pyridylethylrest bedeuten, wobei R⁹ auch Wasserstoff bedeuten kann oder
- R⁸ und R⁹: gemeinsam mit dem dazwischenliegenden Stickstoffatom auch einen gegebenenfalls durch bis zu 4 Methylgruppen substituierten Pyrrolidino-, Piperidino-, Piperazino-, Morpholino- oder Pyrazolinorest bedeuten kann oder
- R⁶ und R⁸: und/oder R⁷ und R⁹ gemeinsam eine solche Brücke bedeuten, daß zusammen mit dem Benzolring, an den R⁶ und/oder R⁷ gebunden sind, und dem Stickstoffatom, an das R⁸ und/oder R⁹ gebunden sind, ein gegebenenfalls durch C₁-C₄-Alkyl substituierter Dihydroindol-, Tetrahydrochinolin-, Tetrahydrochinoxalin-, Tetrahydro-1,4-benzoxazin- oder Julolidinring gebildet wird,
- R¹⁰ und R¹¹: unabhängig voneinander je Wasserstoff, gegebenenfalls durch Hydroxy, Halogen, C₁-C₄-Alkoxy und/oder Cyano substituiertes C₁-C₈-Alkyl, C₂-C₄-Alkenyl, Cyclopentyl, Cyclohexyl, einen gegebenenfalls durch Halogen, Cyano, C₁-C₄-Alkyl und/oder C₁-C₄-Alkoxy substituierten Benzyl- oder Phenylrest, Thienyl oder Pyridyl bedeuten, wobei R¹¹ auch O-C₁- bis C₆-Alkyl, einen gegebenenfalls durch Halogen, Cyano, C₁-C₄-Alkyl und/oder C₁-C₄-Alkoxy substituierten Benzyloxy- oder Phenyloxyrest oder NHR¹⁰ mit R¹⁰ wie hier angegeben bedeuten kann und
- X^{⊖}: ein Anion bedeutet.

Besonders bevorzugt sind kationische 1,3,4-Thiadiazolfarbstoffe der Formel (I), in der
- R¹ und R: unabhängig voneinander Methyl, Ethyl, Propyl, Butyl, Methylpropyl, Pentyl, Methylbutyl, Dimethylpropyl, Hexyl, Hydroxyethyl, Hydroxypropyl, Chlorethyl, Cyanomethyl, Cyanoethyl, Cyanopropyl, Methoxyethyl, Ethoxyethyl, Methoxypropyl, Aminocarbonylmethyl, Aminocarbonylethyl, Methoxycarbonylmethyl, Methoxycarbonylethyl, Allyl, Cyclohexyl, Benzyl, Phenethyl, Methylbenzyl, Chlorbenzyl, Methoxybenzyl, Phenyl, Tolyl, Chlorphenyl, Anisyl, Cyanophenyl, 2- oder 4-Pyridylmethyl oder 2-oder 4-Pyridylethyl bedeuten und R auch Wasserstoff bedeuten kann oder
- R¹ und R: gemeinsam mit dem dazwischenliegenden Stickstoffatom Pyrrolidino, Piperidino, Piperazino oder Morpholino bedeuten,
- R³: Methyl, Ethyl, Propyl, Butyl, Hydroxyethyl, Hydroxypropyl, Hydroxybutyl, Chlorethyl, Cyanomethyl, Cyanoethyl, Cyanopropyl, Methoxyethyl, Ethoxyethyl, Methoxypropyl, Aminocarbonylethyl, Methoxycarbonylethyl, Ethoxycarbonylethyl, Allyl, Benzyl, Phenethyl, Methylbenzyl, Chlorbenzyl oder Methoxybenzyl bedeutet,
- R⁴: Wasserstoff bedeutet,
- R⁵: SO₂R¹⁰ oder COR¹¹ bedeutet,
- R⁶: Wasserstoff, Methyl, Ethyl, Chlor, Methoxy oder Ethoxy bedeutet,
- R⁷: Wasserstoff bedeutet,
- R⁸ und R⁹: unabhängig voneinander Methyl, Ethyl, Propyl, Butyl, Hydroxyethyl, Hydroxypropyl, Hydroxybutyl, Chlorethyl, Cyanomethyl, Cyanoethyl, Cyanopropyl, Methoxyethyl, Ethoxyethyl, C₁-C₂-Alkylcarbonyloxyethyl, Phenylcarbonyloxyethyl, C₁- bis C₂-Alkyloxycarbonyloxyethyl, Cyan-C₁-bis C₂-alkoxyethyl, Chlor-hydroxypropyl, Dihydroxypropyl, Methoxypropyl, Aminocarbonylethyl, Methoxycarbonylethyl, Ethoxycarbonylethyl, Allyl, Benzyl, Phenethyl, Methylbenzyl, Chlorbenzyl, Methoxybenzyl, Phenyl, Methylphenyl, Dimethylphenyl, tert.-Butylphenyl, Chlorphenyl, Dichlorphenyl, Methoxyphenyl, Hydroxymethoxyphenyl, Cyanophenyl, Acetaminophenyl, 2-Thienyl, 2-Tetrahydrofuryl, 2- oder 4-Pyridyl oder 2-oder 4-Pyridylmethyl bedeuten, wobei R⁹ auch Wasserstoff bedeuten kann oder
- R⁸ und R⁹: gemeinsam mit dem dazwischenliegenden Stickstoffatom einen unsubstituierten Pyrrolidino-, Piperidino- oder Morpholinorest bedeuten oder
- R⁶ und R⁸: gemeinsam eine solche Brücke bedeuten, daß zusammen mit dem Benzolring, an den R⁶ gebunden ist, und dem Stickstoffatom, an das R⁸ gebunden ist, ein Dihydroindol-, Methyldihydroindol-, Tetrahydrochinolin-, Methyltetrahydrochinolin-, Trimethyltetrahydrochinolin- oder Tetrahydro-1,4-benzoxazin-Ring gebildet wird,
- R¹⁰ und R¹¹: unabhängig voneinander je Wasserstoff, Methyl, Ethyl, Propyl, Butyl, Hydroxyethyl, Hydroxypropyl, Hydroxybutyl, Cyanoethyl, Methoxyethyl, Vinyl, Allyl, Methylvinyl, Cyclohexyl, Benzyl, Chlorbenzyl, Phenyl, Methylphenyl, Dimethylphenyl, Chlorphenyl, Methoxyphenyl, 2-Thienyl oder 2-, 3-oder 4-Pyridyl bedeuten und
- X^{⊖}: ein farbloses Anion bedeutet.

Ganz besonders bevorzugt sind kationische 1,3,4-Thiadiazolfarbstoffe der Formel (I), in der
- R¹ und R: unabhängig voneinander Methyl, Ethyl, Propyl, Butyl, Hydroxyethyl, Hydroxypropyl, Cyanoethyl, Methoxyethyl oder Ethoxyethyl bedeuten oder R¹ und R gemeinsam mit dem dazwischenliegenden Stickstoffatom Morpholino bedeuten,
- R³: Methyl, Ethyl, Hydroxyethyl, Hydroxypropyl oder Cyanoethyl bedeutet,
- R⁴: Wasserstoff bedeutet,
- R⁵: COR¹¹ bedeutet,
- R⁶: Methyl, Chlor oder Methoxy bedeutet,
- R⁷: Wasserstoff bedeutet,
- R⁸: Phenyl, Methylphenyl, Dimethylphenyl, tert.-Butylphenyl, Chlorphenyl, Dichlorphenyl, Methoxyphenyl, Hydroxyethoxyphenyl, Cyanophenyl oder Acetaminophenyl bedeutet,
- R⁹: Wasserstoff bedeutet,
- R¹¹: Methyl oder Ethyl bedeutet und
- X^{⊖}: ein farbloses Anion bedeutet.

Die vorliegende Erfindung betrifft weiterhin Anhydrobasen der Formeln (II) und (III), 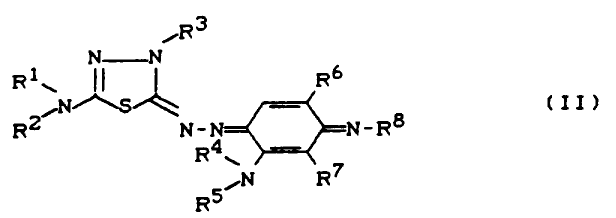 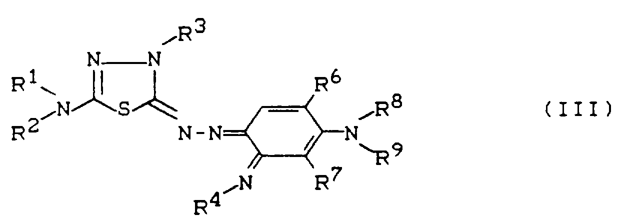 in denen R¹ bis R⁹ die bei Formel (I) angegebene allgemeine, beispielsweise, vorzugsweise, besonders vorzugsweise und ganz besonders vorzugsweise Bedeutung haben.

Die Anhydrobasen der Formeln (II) und (III) können aus den Farbstoffen der Formel (I) durch Umsetzung mit Basen in Gegenwart eines Lösungsmittels erhalten werden.

Geeignete Basen sind beispielsweise Hydroxide wie Natrium-, Kalium- und Calciumhydroxid, Oxide wie Magnesiumoxid, Alkoholate wie Natriummethylat, -ethylat oder -tert.-butylat, Amine wie Triethylamin, Di- oder Triethanolamin, Piperidin oder Pyridin oder basische Ionenaustauscher auf Basis Styrol/Divinylbenzol.

Geeignete Lösungsmittel sind beispielsweise Wasser, Alkohole wie Methanol, Ethanol, Isopropanol oder Glykole, Ketone wie Aceton oder Butanon, Amide wie Dimethylformamid, Dimethylacetamid oder N-Methyl-pyrrolidon, Nitrile wie Acetonitril und 3-Hydroxy-propionitril, Sulfoxide wie Dimethyl-sulfoxid, Sulfone wie Sulfolan und Dimethylsulfon oder N-Methylcaprolactam oder Mischungen davon.

Die Anhydrobasen der Formeln (II) und (III) sind geeignete Zwischenprodukte zur Herstellung von Farbstoffen der Formel (I) mit solchen Anionen X^{⊖}, die durch eines der nachfolgend beschriebenen Herstellungsverfahren für Farbstoffe der Formel (I) nicht oder nur mit großem Aufwand eingeführt werden können. Man kann so Farbstoffe der Formel (I) herstellen, die durch Variation des Anions X^{⊖} besondere Eigenschaften erhalten, z.B. bessere Löslichkeit und/oder bessere Eignung zur Herstellung von flüssigen Formulierungen. Hierzu können Anhydrobasen der Formeln (II) oder (III) mit der Säure der Formel HX umgesetzt werden, deren Anion eingeführt werden soll. Die Umsetzung kann gegebenenfalls in Gegenwart eines Lösungsmittels und unter Kühlung, bei Raumtemperatur oder Temperaturen bis zum Siedepunkt des Mediums durchgeführt werden.

Lösungsmittel können beispielsweise sein ein Überschuß an der Säure HX, Wasser, Glykole wie Ethylen- oder Propylenglykol, Amide wie c-Caprolactam, Nitrile wie Oxypropionitril, die oben für die Herstellung der Anhydrobasen aufgeführten Lösungsmittel oder Mischungen davon.

So hergestellte Farbstoffe der Formel (I) fallen entweder aus dem Reaktionsgemisch aus oder ergeben darin eine stabile Lösung.

Die Anhydrobasen der Formeln (II) und (III) selbst sind auch geeignet zum Anfärben von Fasern und Geweben aus Polyester sowie als Farbstoffe für den Sublimationstransferdruck, wie er beispielsweise in EP-A 0 384 040 beschrieben ist.

Die vorliegende Erfindung betrifft weiterhin ein Verfahren zur Herstellung von kationischen 1,3,4-Thiadiazolfarbstoffen der Formel (I), das dadurch gekennzeichnet ist, daß man 2-Amino-1,3,4-thiadiazole der Formel (IV) 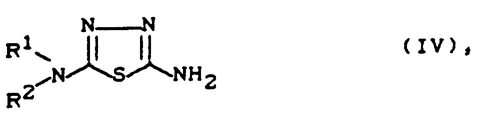 in der
R¹ und R die im Anspruch 1 angegebene Bedeutung haben, auf m-Phenylendiaminderivate der Formel (V) 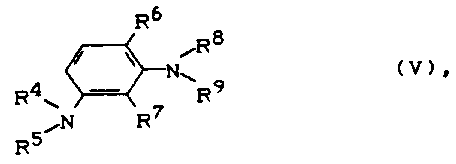 in der
R⁴ bis R⁹ die im Anspruch 1 angegebene Bedeutung haben, kuppelt und dann mit Verbindungen der Formel (VI)

(R³)X (VI),

in der
R³ und X die in Anspruch 1 angegebene Bedeutung haben, quarterniert.

Die 2-Amino-1,3,4-thiadiazole der Formel (IV) sind z.B. aus der DE-A 2 811 258 bekannt oder analog dazu erhältlich. Die m-Phenylendiaminderivate der Formel (V) sind z.B. aus den DE-A 3 840 065, 3 901 839 und 3 906 189 bekannt oder analog dazu erhältlich.

Die vorliegende Erfindung betrifft noch ein weiteres Verfahren zur Herstellung von kationischen 1,3,4-Thiadiazolfarbstoffen der Formel (I), das dadurch gekennzeichnet ist, daß man Farbstoffe der Formel (VII) 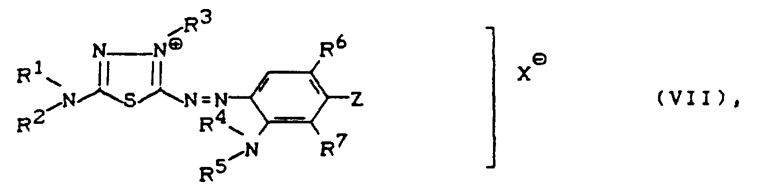 in der
- R¹ bis R⁷: und X^{⊖} die im Anspruch 1 angegebene Bedeutung haben und
- Z: für Halogen, Hydroxy, Alkoxy, Cycloalkoxy, Aryloxy, Amino oder Dialkylamino steht, mit Aminen der Formel (VIII) 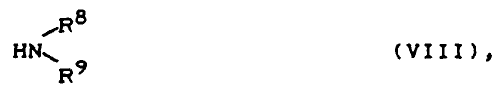
in der
R⁸ und R⁹ die in Anspruch 1 angegebene Bedeutung haben, umsetzt.

In Formel (VII) steht Z vorzugsweise für Fluor, Chlor, Brom, Hydroxy, C₁-C₄-Alkoxy, C₅-C₇-Cycloalkoxy, C₆-C₁₀-Aryloxy oder NR'R", wobei R' und R" unabhängig voneinander für Wasserstoff oder C₁-C₆-Alkyl stehen.

Verbindungen der Formel (VII) sind neu und können beispielsweise erhalten werden, indem man 2-Amino-1,3,4-thiadiazole der Formel (IV) auf ein m-Phenylendiaminderivat der Formel (IX) 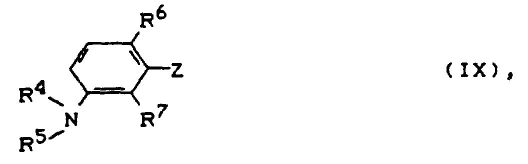 in der
- R⁴ bis R⁷: die in Anspruch 1 und Z die in Anspruch 8 angegebene Bedeutung haben,
kuppelt und dann mit Verbindungen der Formel (VI) quaterniert.

Die erwähnten Diazotierungen können in an sich bekannter Weise durchgeführt werden, beispielsweise mit Nitrosylschwefelsäure in 80 bis 90 gew.-%iger Phosphorsäure oder in Gemischen solcher Phosphorsäuren mit Essigsäure, Propionsäure und/oder Schwefelsäure. Die erwähnten Kupplungen können ebenfalls auf an sich bekannte Weise durchgeführt werden, beispielsweise in einem sauren Medium, das wäßrig oder wäßrig-organisch sein kann.

Die Diazotierungen und Kupplungen können auch nach anderen an sich bekannten Verfahren simultan durchgeführt werden, beispielsweise indem man Verbindungen der Formeln (IV) und Verbindungen der Formeln (V) oder (IX) gemeinsam in einem sauren Medium mit beispielsweise Natriumnitrit umsetzt. Als saure Medien sind z.B. wäßrige Mineralsäuren oder organische Säuren oder Mischungen davon geeignet, wobei als Mineralsäuren z.B. Salzsäure, Schwefelsäure oder Phosphorsäure und als organische Säuren z.B. Ameisensäure, Essigsäure oder Propionsäure in Frage kommen. Auch unter Druck verflüssigtes Kohlendioxid kann als saures Medium dienen.

Als quaternierende Mittel kommen z.B. in Betracht Alkylhalogenide, Halogenacetamide, β-Halogenpropionitrile, Halogenhydrine, Alkylenoxide, Alkylester der Schwefelsäure, Alkylester organischer Sulfonsäuren, Nitrile, Amide und Ester von α,β-ungesättigten Carbonsäuren, Alkoxyalkylhalogenide und Vinylpyridine. Als Beispiele seien genannt: Methylchlorid, Methylbromid, Methyliodid, Benzylchlorid, Benzylbromid, Chloracetamid, β-Chlorpropionitril, Ethylenchlorhydrin, Dimethylsulfat, Diethylsulfat, Benzolsulfonsäuremethylester, Benzolsulfonsäureethyl-ester, Toluolsulfonsäuremethylester, Toluolsulfonsäureethylester, Toluolsulfonsäurepropylester, Allylchlorid, Allylbromid, Ethylenoxid, Propylenoxid, Acrylnitril, Acrylsäure, Acrylamid, Acrylsäuremethylester, 2- und 4-Vinylpyridin, Sulfolen (= 1,1-Dioxo-2,5-dihydrothiophen), Epichlorhydrin, Styroloxid, Methylphosphonsäuredimethylester und Allylphosphorsäureester.

Die erwähnten Quaternierungen können z.B. in einem indifferenten organischen Lösungsmittel, in Wasser oder in Mischungen davon erfolgen, wobei gegebenenfalls säurebindende Mittel, wie Magnesiumoxid, Natriumcarbonat, Natriumhydrogencarbonat, Calciumcarbonat oder Natriumacetat, zugesetzt werden können. Geeignete organische Lösungsmittel sind beispielsweise Kohlenwasserstoffe, Chlorkohlenwasserstoffe, Nitrokohlenwasserstoffe, Nitrile, Amide, Carbonsäuren, Carbonsäureanhydride, Ketone und Dialkylsulfoxide, wie Benzol, Toluol, Tetrachlorethan, Mono- und Dichlorbenzol, Nitrobenzol, Acetonitril, Propionitril, Dimethylformamid, N-Methylpyrrolidon, Essigsäure, Propionsäure, Milchsäure, Acetanhydrid, Aceton, Butanon und Dimethylsulfoxid. Die Umsetzung der Farbstoffe der Formel (VII) mit Aminen der Formel (VIII) kann ebenfalls in organischen Lösungsmitteln, in Wasser oder in Mischungen davon erfolgen. Geeignete Lösungsmittel sind die oben beschriebenen. Die Umsetzung kann gegebenenfalls mit einem Überschuß des Amins der Formel (VI) bei Temperaturen von z.B. zwischen 0 und 100°C, vorzugsweise solchen zwischen 10 und 50°C durchgeführt werden.

Die entstehenden Farbstoffe der Formel (I) fallen entweder aus den Lösungsmitteln direkt aus und können z.B. durch Abfiltrieren isoliert werden oder man kann sie bei der Verwendung von mit Wasser mischbaren Lösungsmitteln als feste, abfiltrierbare Produkte erhalten durch Verdünnen mit Wasser und Zugabe wasserlöslicher Salze wie Natrium- oder Kaliumchlorid, gegebenenfalls in Gegenwart von Zinkchlorid.

Die erfindungsgemäßen Farbstoffe der Formel (I) und die Farbstoffe der Formel (VII) eignen sich hervorragend zum Färben und Bedrucken von kationisch färbbaren Fasern, vorzugsweise von Polymerisaten und Mischpolymerisaten des Acrylnitrils und Dicyanoethylens, sowie von sauer modifizierten Fasern aus Polyamid und Polyester, wobei echte Farbtöne erhalten werden. Die Farbstoffe können auch Verwendung finden zum Färben und Bedrucken von tannierten Zellulosematerialien, Papier, Seide und Leder. Sie sind weiter geeignet zur Herstellung von Schreibflüssigkeiten, Stempelflüssigkeiten und Kugelschreiberpasten und lassen sich auch im Gummidruck verwenden.

Das Färben von beispielsweise Polymerisaten und Mischpolymerisaten des Acrylnitrils kann z.B. aus schwach saurer Flotte erfolgen, wobei man in das Färbebad vorzugsweise bei 40 bis 60°C eingeht und dann bei Kochtemperatur färbt. Man kann auch unter Druck bei Temperaturen über 100°C färben. Des weiteren lassen sich mit den erfindungsgemäßen Farbstoffen Spinnlösungen zum Färben polyacrylnitrilhaltiger Fasern herstellen,

Die Färbungen der erfindungsgemäßen Farbstoffe der Formel (I) auf Materialien aus Polyacrylnitril zeichnen sich durch sehr gute Licht-, Naß- und Reibechtheiten und durch eine hohe Affinität zur Faser auf.

Erfindungsgemäße Farbstoffe können einzeln, in Mischungen untereinander oder in Mischungen mit anderen Farbstoffen angewendet werden.

Die vorliegende Erfindung betrifft schließlich auch kationisch färbbare Fasern, tannierte Zellulosematerialien, Papier, Seide, Leder, Kugelschreiberpasten, Schreibflüssigkeiten und Stempelflüssigkeiten, die dadurch gekennzeichnet sind, daß sie mindestens einen kationischen 1,3,4-Thiadiazolfarbstoff des Anspruchs 1 enthalten.

### Beispiele

### Beispiel 1

41,7 g 2-Amino-5-diisopropylamino-1,3,4-thiadiazol (96 gew.-%ig) und 42,05 g 3-Acetylamino-N,N-diethylanilin wurden in 200 ml Eisessig gelöst. Bei 25°C wurde während 90 Minuten eine Lösung von 13,8 g Natriumnitrit in 24 g Wasser zugetropft und anschließend über Nacht bei Raumtemperatur gerührt. Dann wurden bei 35 bis 40°C während 3 Stunden 41,8 g Dimethylsulfat zugetropft, gefolgt vom Zusatz von 16,2 g wasserfreiem Natriumacetat. Nach 2 Stunden bei 40°C wurde über Nacht bei Raumtemperatur gerührt, auf 500 ml Wasser ausgetragen und filtriert. Dem Filtrat wurden 100 ml 2-molare Zinkchloridlösung und 450 ml gesättigte Natriumchloridlösung zugesetzt. Über Nacht wurde gerührt, abgesaugt und im Vakuum getrocknet. Es wurden 112,6 g blaue Kristalle der Formel (I) mit R¹ = R = Isopropyl, R³ = CH₃, R⁴ = R⁶ = R⁷ = H, R⁵ = COCH₃, R⁸ = R⁹ = C₂H₅ und X^{⊖} = ZnCl₃ ⁻ erhalten. λ_{maX}, gemessen in 10 %iger Essigsäure, betrug 596 nm.

Der so erhaltene Farbstoff färbte Polyacrylnitril in einem leicht rotstichigen Blau.

### Beispiel 2

a) 20,9 g 2-Amino-5-diisopropylamino-1,3,4-thiadiazol (96 gew.-%ig) wurden in einem Gemisch aus 50 ml Eisessig, 15 g 48 gew.-%iger Schwefelsäure und 5 g 85 gew.-%iger Phosphorsäure gelöst. Bei -5°C wurden während 45 Minuten 34 g 40 gew.-%ige Nitrosylschwefelsäure (in Schwefelsäure) dazugetropft.
b) 15,3 g 4-Aminoveratrol wurden in 100 ml Eisessig gelöst und 10,2 g Acetanhydrid zugetropft. Danach wurde 1 Stunde bei Raumtemperatur nachgerührt.
c) Zu der Lösung von 4-Acetaminoveratrol aus b) wurde bei 0°C die Diazolösung aus a) zulaufen gelassen und danach 2 Stunden bei Raumtemperatur nachgerührt.
d) 32,8 g wasserfreies Natriumacetat wurde zugesetzt und dann bei 40°C 30,4 ml Dimethylsulfat während 3 Stunden dazugetropft, 8,2 g wasserfreies Natriumacetat wurde zugesetzt und 2 Stunden bei 40°C nachgerührt.
e) Bei Raumtemperatur wurden 18,6 g Anilin zugegeben und der pH-Wert auf 3,7 eingestellt. Nach Rühren über Nacht wurden 100 ml gesättigte Natriumchloridlösung zugesetzt. Es wurde abgesaugt und dann mit Natriumchloridlösung gewaschen. Nach dem Trocknen wurden 49,7 g eines blauen Pulvers der Formel (I) mit R¹ = R = Isopropyl, R³ = CH₃, R⁴ = R⁶ = R⁷ = R⁸ = H, R⁵ = COCH₃, R⁹ = Phenyl und X^{⊖} = Cl^{⊖} erhalten. Der λₘₐₓ-Wert, gemessen in 10 Gew.-%iger Essigsäure, betrug 616 nm.

Der Farbstoff färbte Polyacrylnitril und holzschliffhaltiges Papier in einem grünstichigen Blau.

Analog den Beispielen 1 und 2 wurden die folgenden Beispiele durchgeführt (siehe Tabellen 1 bis 5):

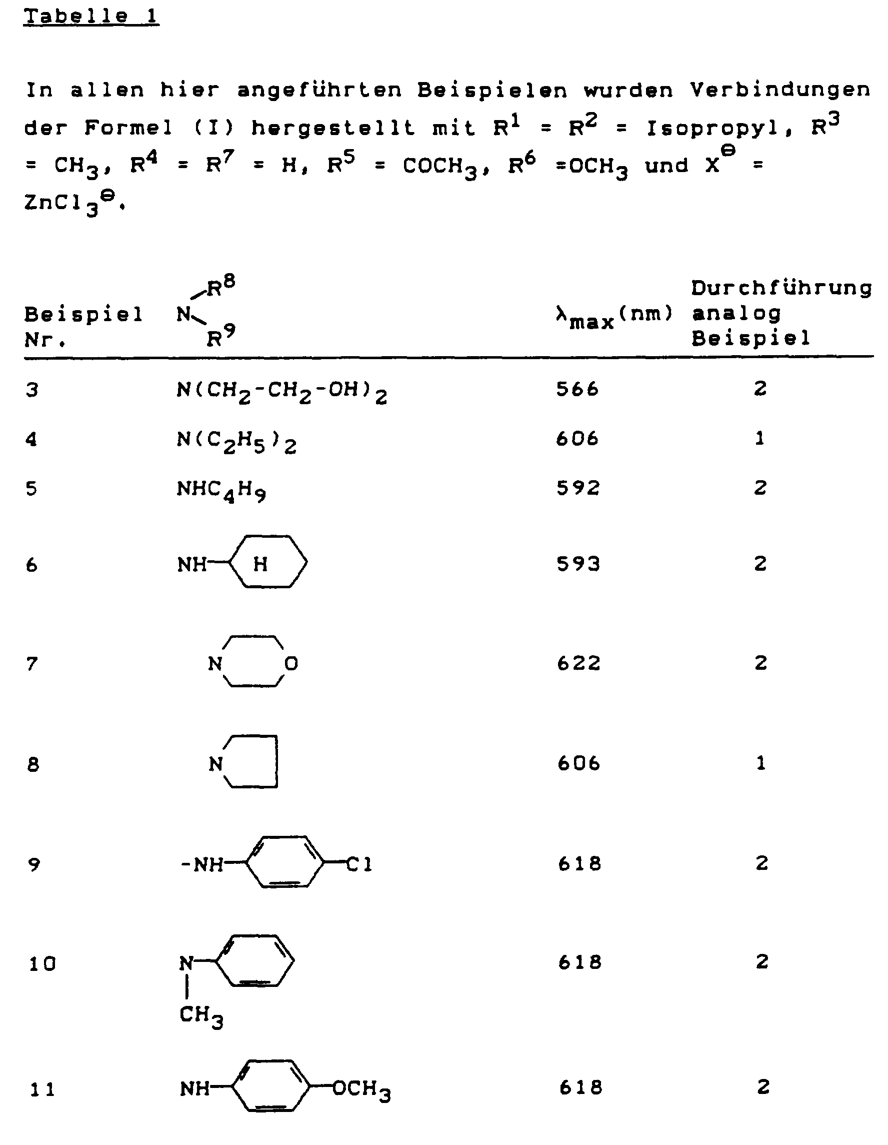

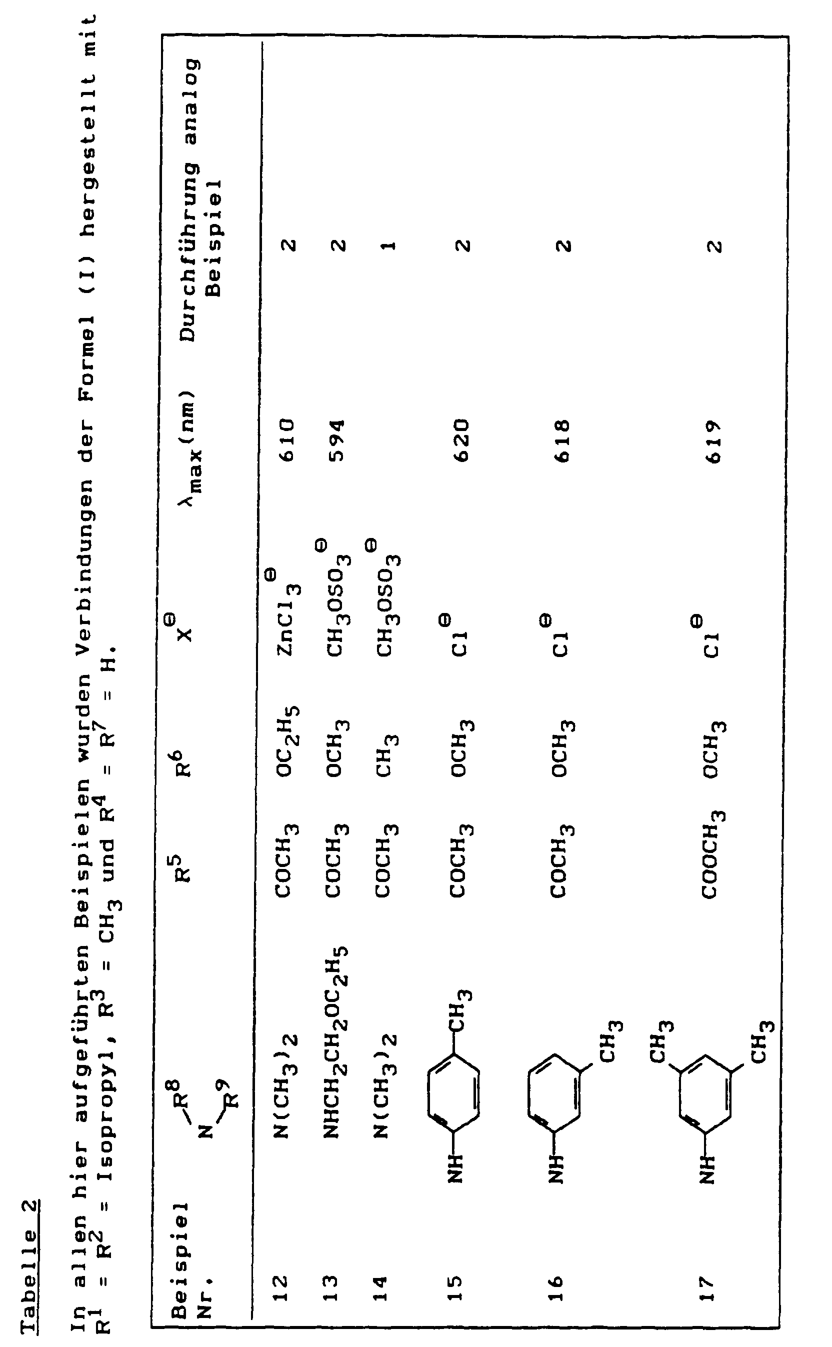

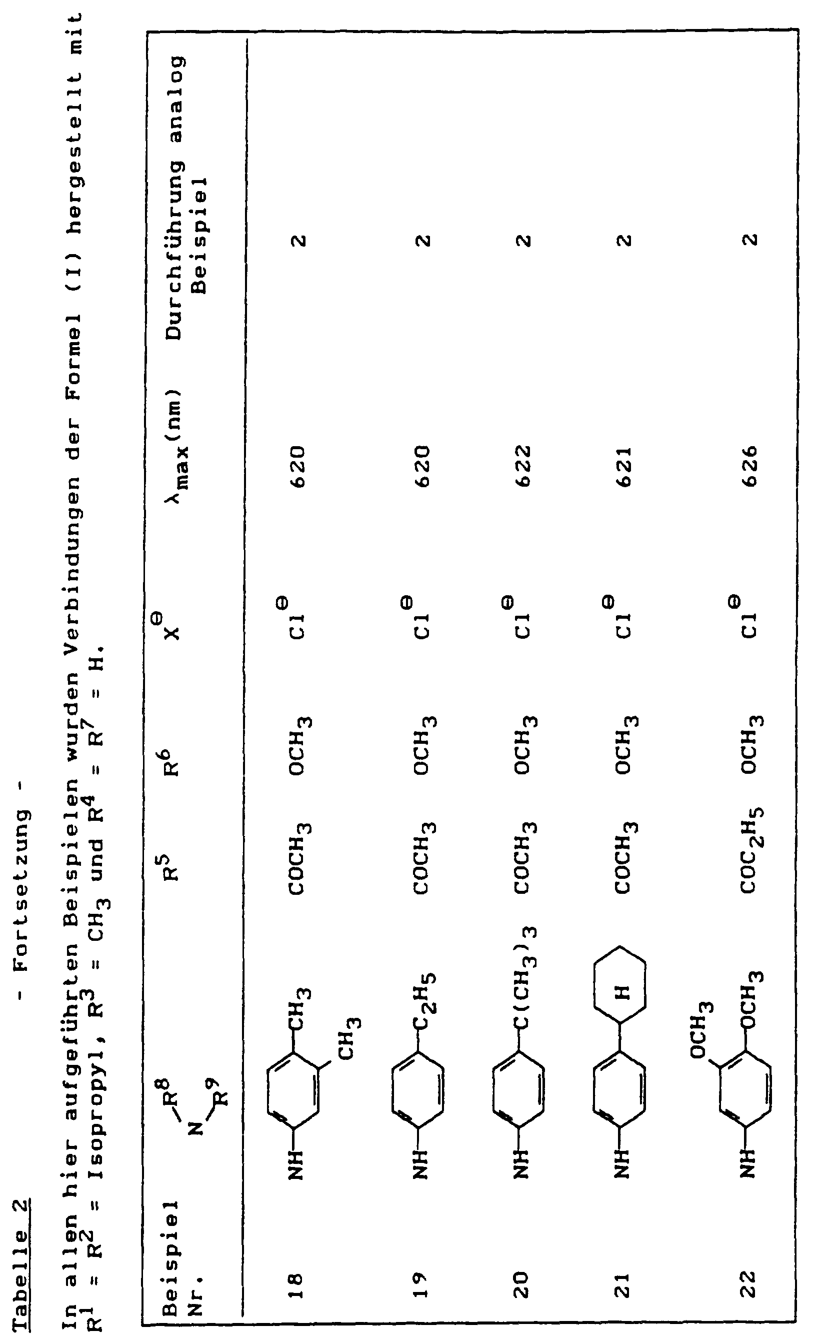

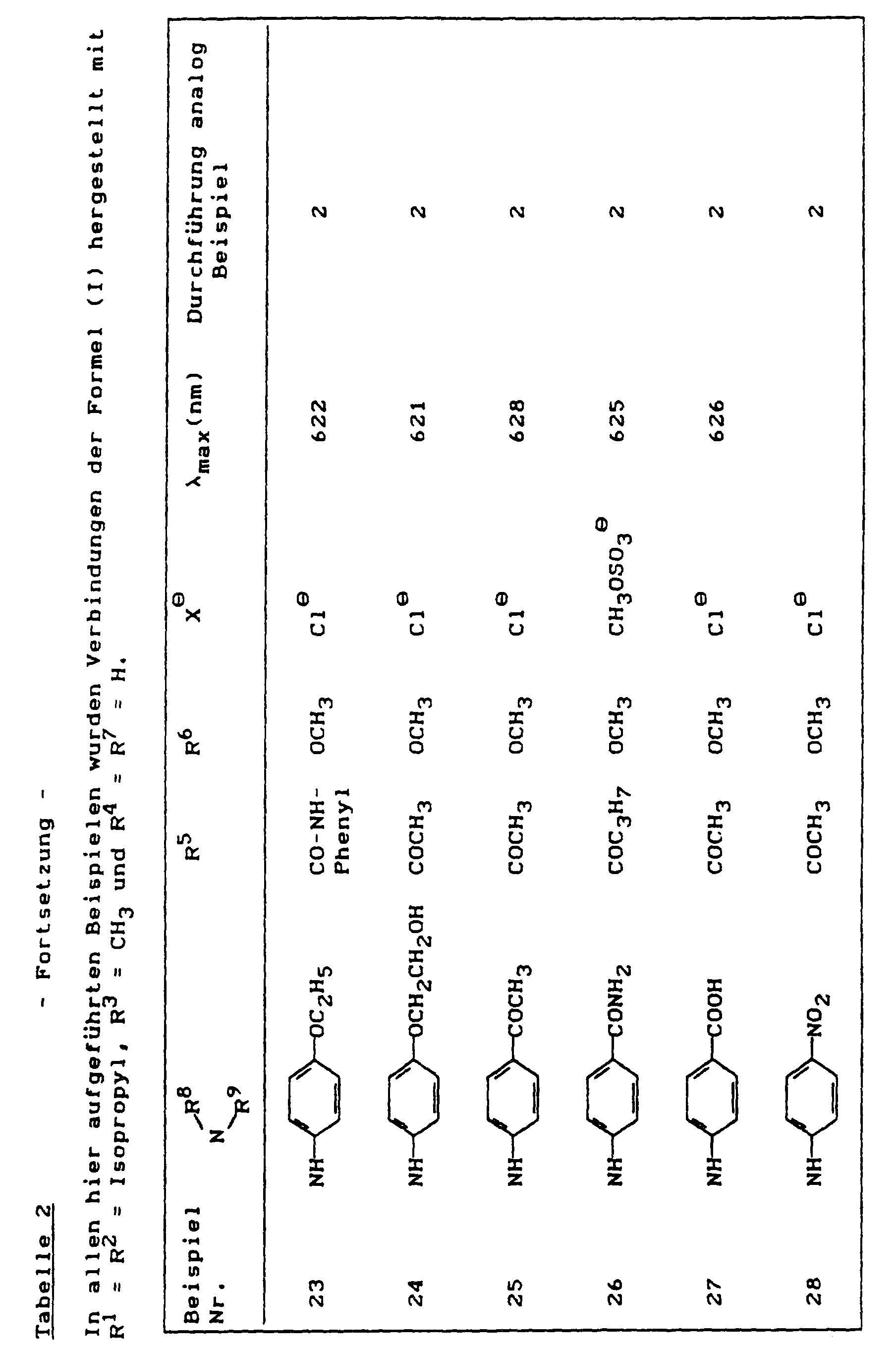

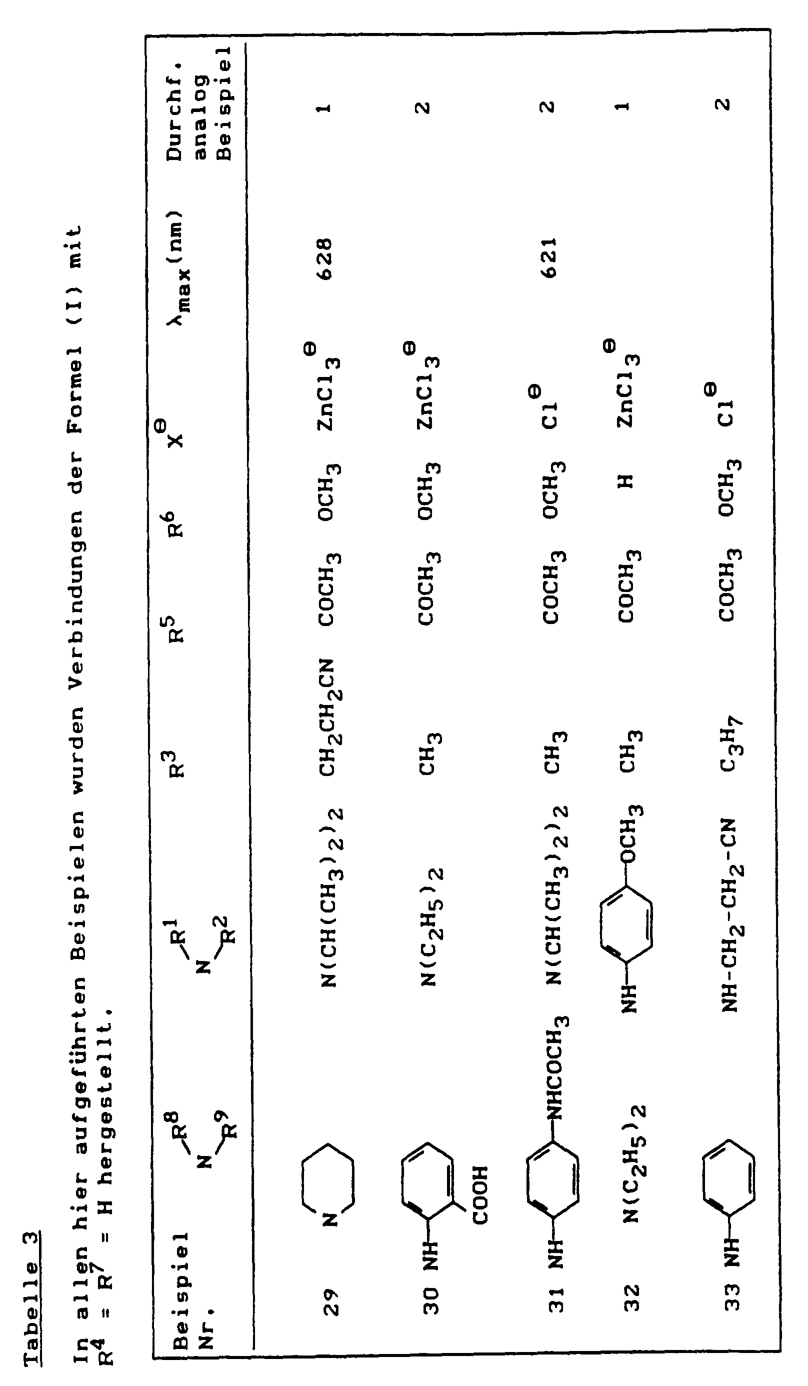

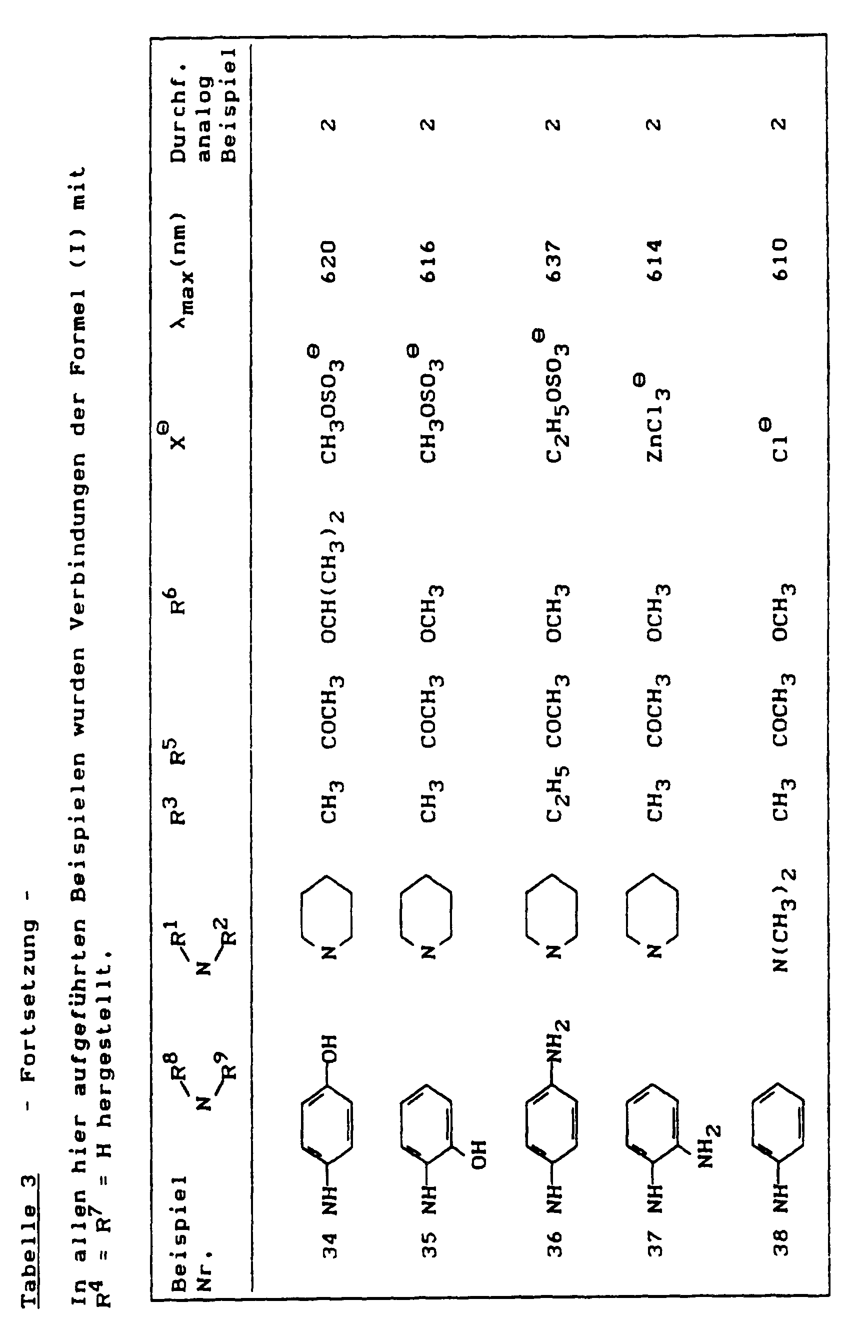

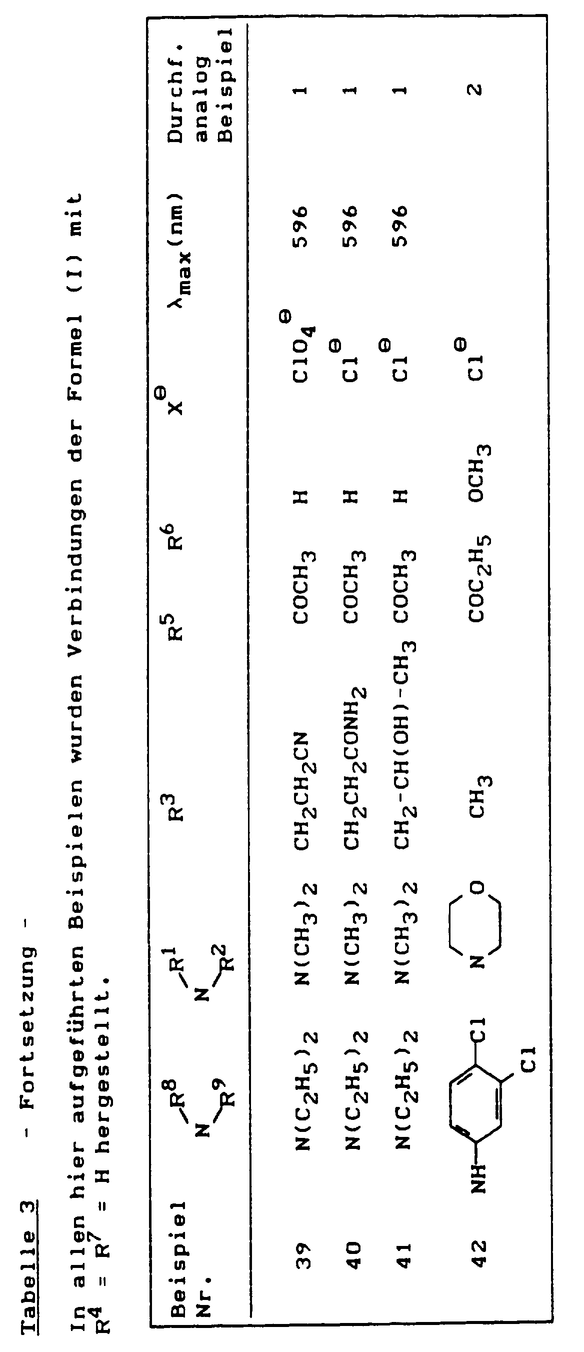

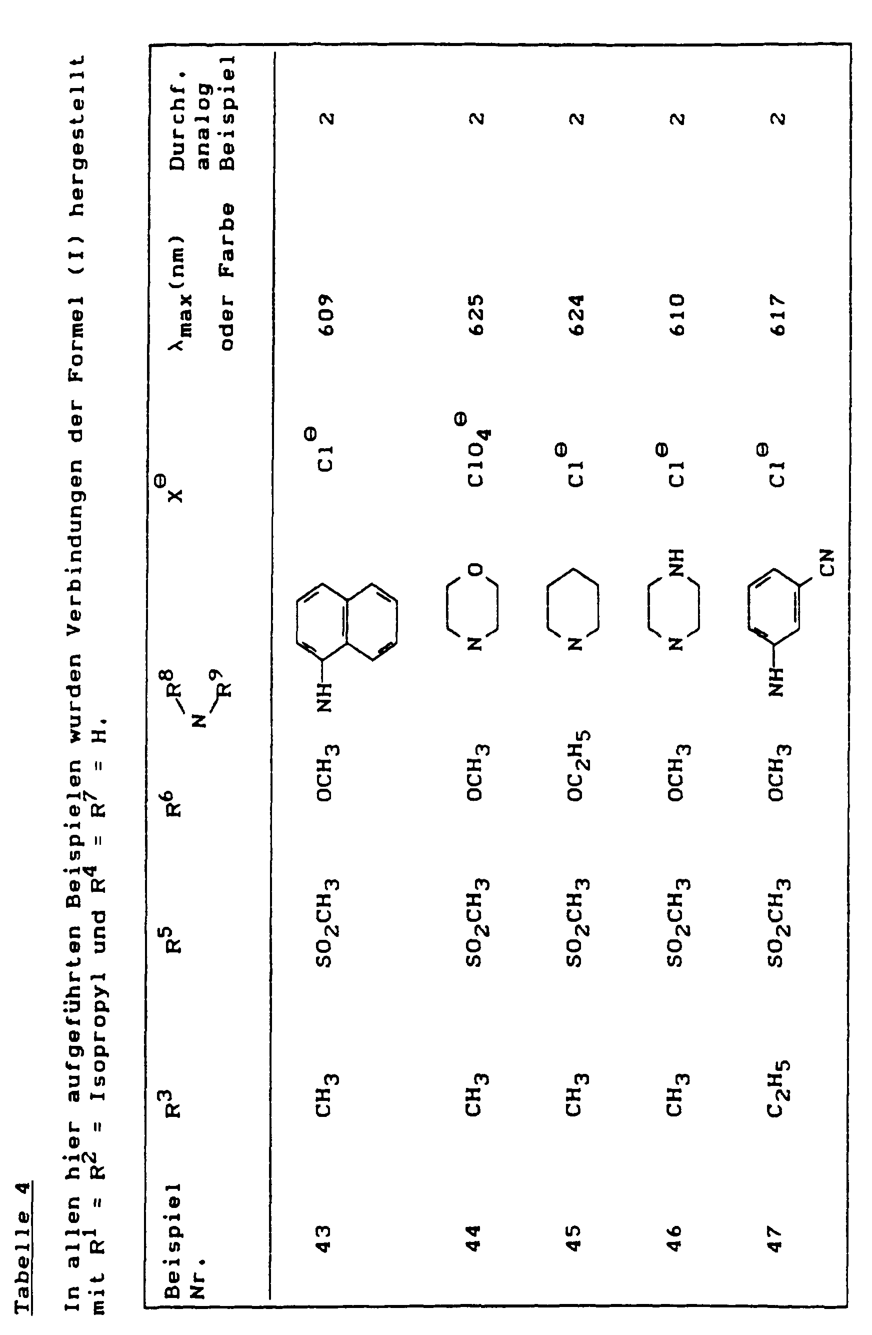

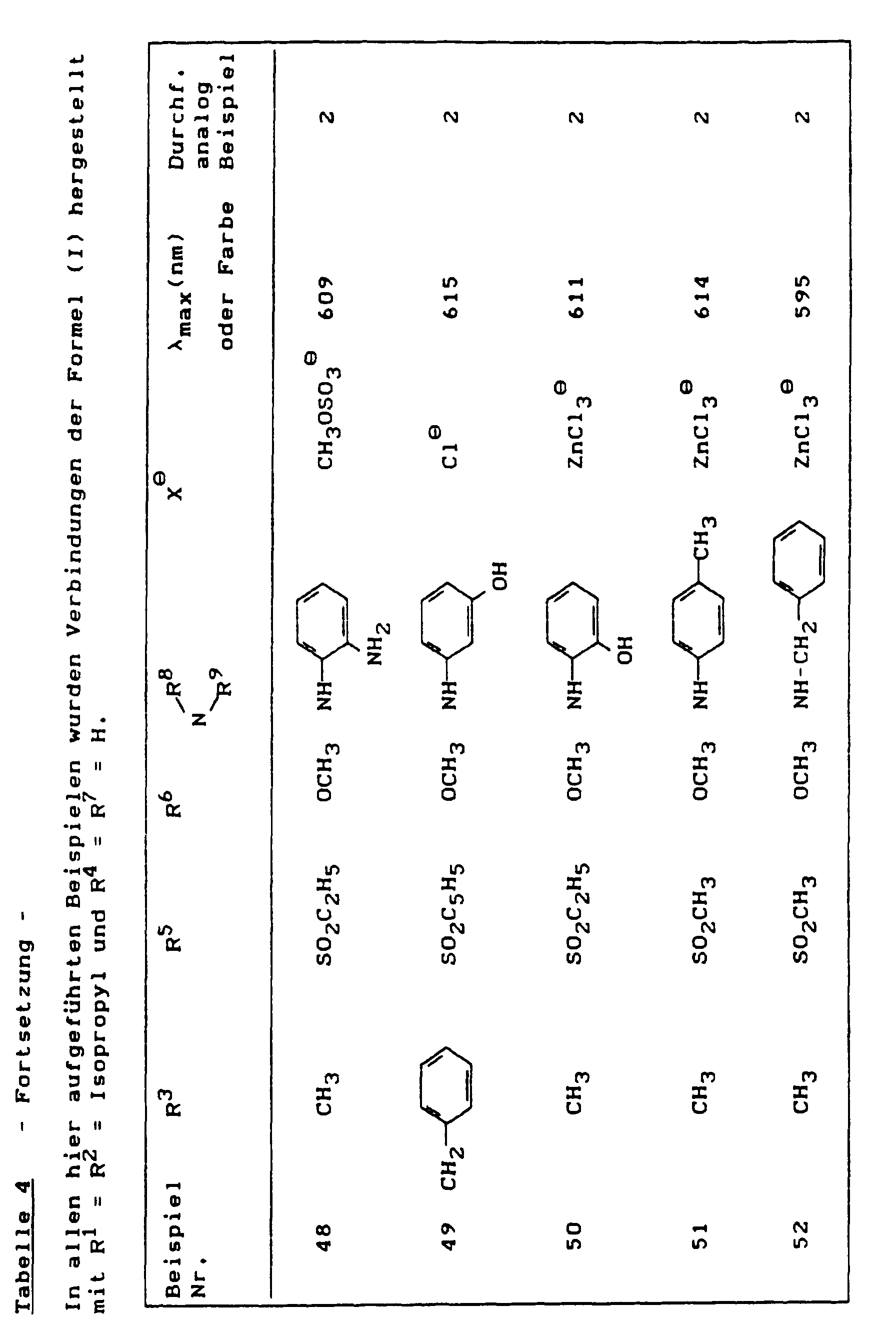

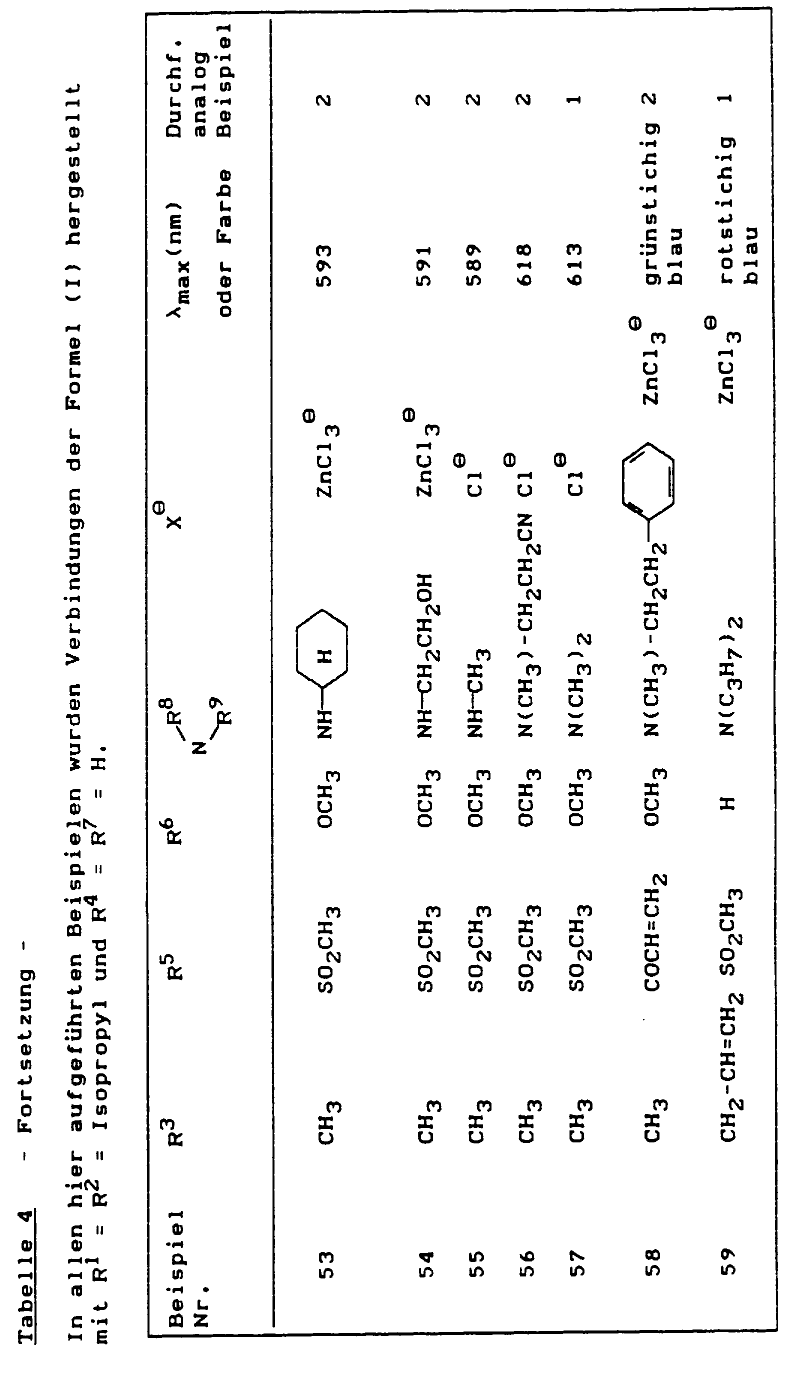

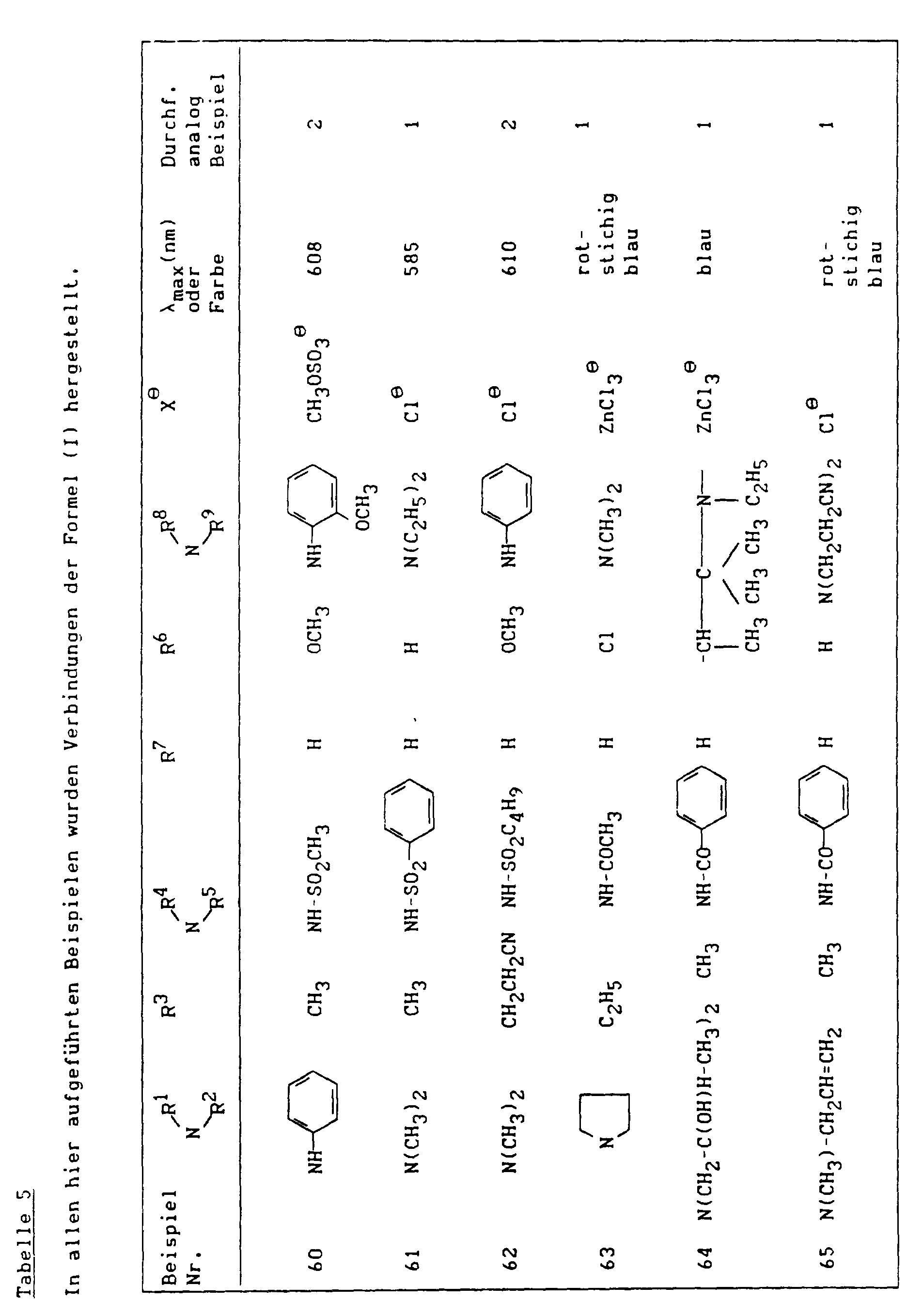

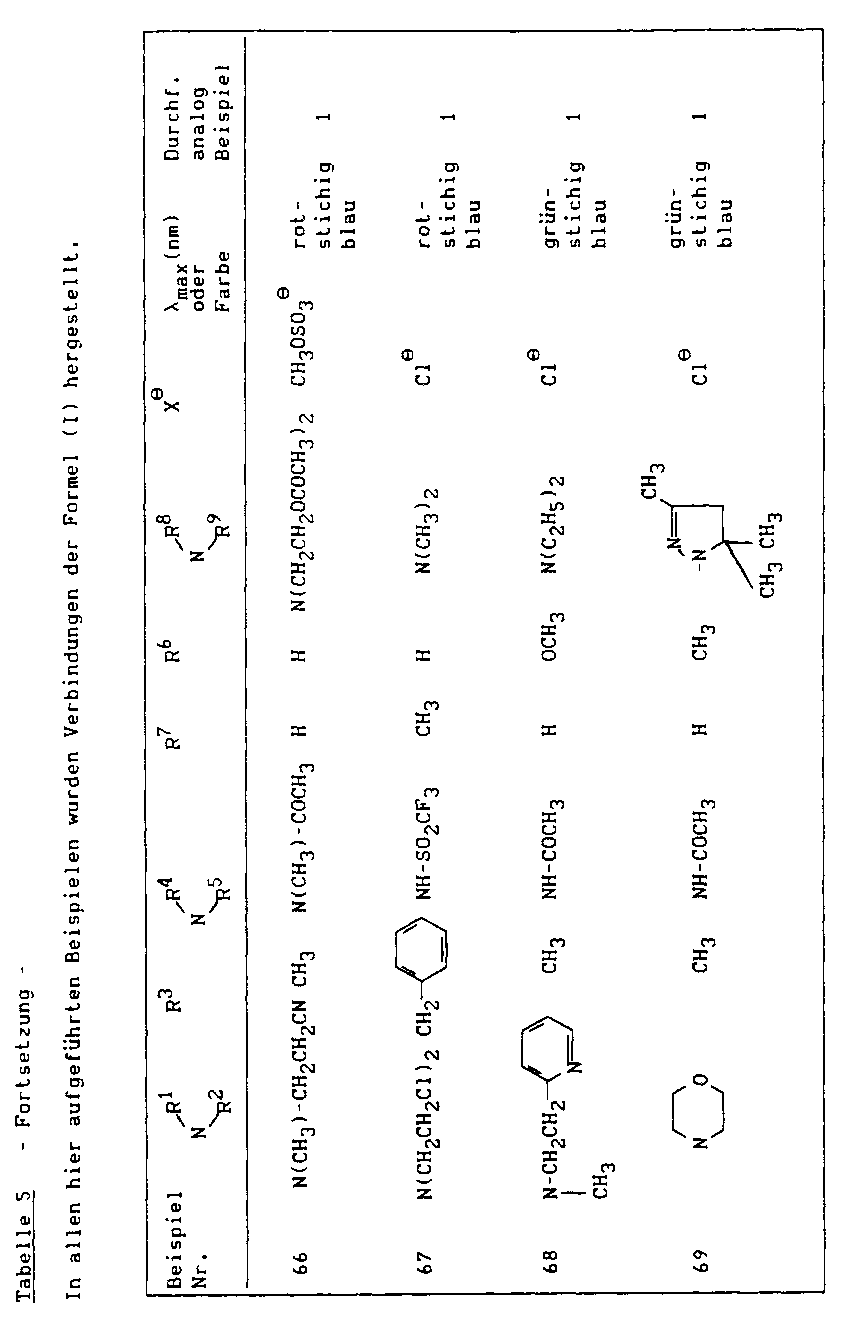

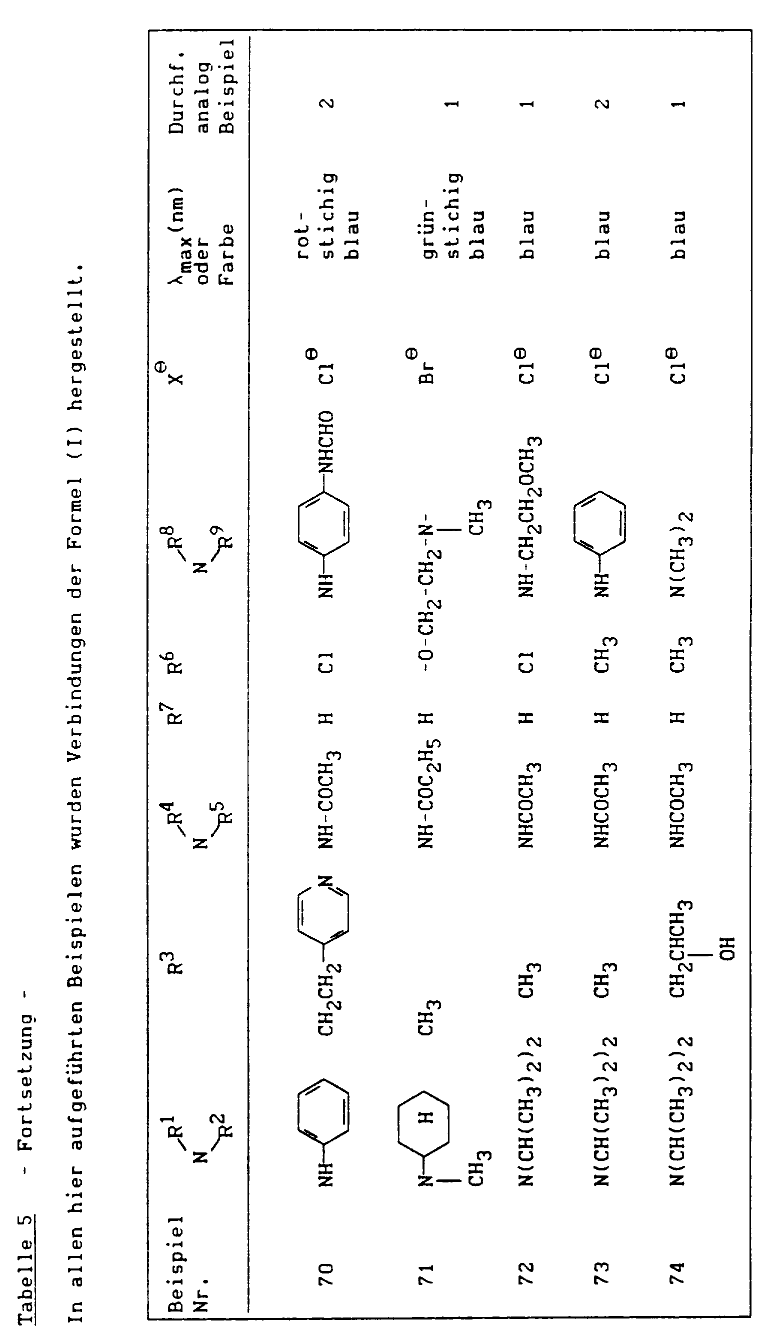

### Beispiel 75

### Herstellung einer flüssigen Farbstoffzubereitung

10 g des Farbstoffs des Beispiels 2 wurden in 140 ml Methanol gelöst und so lange 10-%ige Natronlauge zugetropft, bis die blaue Farbe der Lösung verschwunden war. Die ausgefallenen roten Schuppen der Formel (II) mit R¹ = R = Isopropyl, R³ = CH₃, R⁴ = R⁷ = H, R⁵ = COCH₃, R⁶ = OCH₃ und R⁸ = Phenyl wurden abgesaugt und getrocknet. Die Ausbeute betrug 6,7 g = 80 % der Theorie, der Schmelzpunkt 208-210°C, λₘₐₓ gemessen in Dimethylformamid 509 nm.

4,8 g dieser Verbindung wurden in einer Mischung aus 3,2 g Eisessig, 2,0 g Milchsäure und 9,0 g Wasser bei 90°C gelöst. Nach dem Abkühlen wurde eine stabile tiefblaue Lösung erhalten, die 30 Gew.-% des Farbstoffs der Formel (I) mit R¹ = R = Isopropyl, R³ = CH₃, R⁴ = R⁷ = R⁹ = H, R⁵ = COCH₃, R⁶ = OCH₃, R⁸ = Phenyl und X^{⊖} = CH₃C(OH)H-COO^{⊖} enthielt.

### Beispiel 76

### Färbeverfahren für Polyacrylnitril

0,1 g des gemäß Beispiel 2 erhaltenen Farbstoffs wurden mit 2 ml Wasser unter Zusatz von wenig Essigsäure angeteigt und mit 50 ml heißem Wasser gelöst. Dann wurden 1,2 g eines Kondensationsprodukts aus Naphthalinsulfonsäure und Formaldehyd zugesetzt und mit kaltem Wasser auf 500 ml aufgefüllt.

Der pH-Wert dieser Färbeflotte wurde mit Essigsäure und Natriumacetat auf 4,5 - 5 eingestellt. In dieser Färbeflotte wurden 10 g Stückware aus Polyacrylnitrilfasern ständig in Bewegung gehalten, während in 30 min die Temperatur auf 100°C erhöht wurde. Bei Kochtemperatur wurde 60 min gefärbt, dann das Material mit kaltem Wasser gespült und bei 60-70°C getrocknet.

### Beispiel 77

### Färbeverfahren für holzschliffhaltiges Papier

Ein aus 60 % Holzschliff und 40 % ungebleichtem Sulfitzellstoff bestehender Trockenstoff wurde im Holländer mit soviel Wasser angeschlagen und bis zum Mahlgrad 40° SR gemahlen, daß der Trockengehalt etwas über 2,5 % lag. Anschließend wurde mit Wasser exakt 2,5 % Trockengehalt des Dickstoffs eingestellt. 200 g dieses Dickstoffs wurden mit 5 g einer 0,5 gew.-%igen wäßrigen Lösung des Farbstoffs des Beispiels 15 versetzt, 5 min verrührt, 2 % Harzleim und 4 % Alaun, bezogen auf Trockenstoff, hinzugegeben und wiederum einige Minuten homogen verrührt. Dann wurde die Masse mit Wasser auf 700 ml verdünnt und hieraus in bekannter Weise durch Absaugen über einen Blattbildner Papierblätter hergestellt. Sie wiesen eine intensive grünstichig blaue Färbung auf.

Analog Beispiel 76 und 77 können die anderen Farbstoffe der Beispiele 1-75 eingesetzt werden.

## Patentansprüche

1. Kationische 1,3,4-Thiadiazolfarbstoffe der Formel (I) 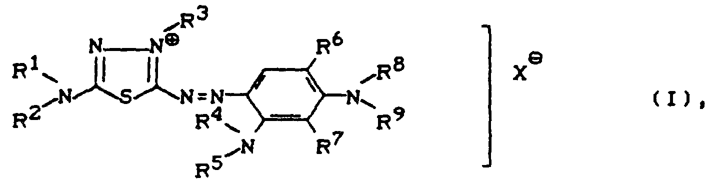 in der
R¹ und R unabhängig voneinander Wasserstoff, Alkyl, Alkenyl, Cycloalkyl, Aralkyl, Aryl, einen gegebenenfalls über eine Methylen- oder Ethylenbrücke gebundenen Heterocyclus oder R¹ und R gemeinsam mit dem dazwischenliegenden Stickstoffatom einen Heterocyclus bedeuten,
R³ Alkyl, Alkenyl oder Aralkyl bedeutet,
R⁴ Wasserstoff oder Alkyl bedeutet,
R⁵ eine Acylgruppe bedeutet,
R⁶ und R⁷ unabhängig voneinander Wasserstoff, Alkyl, Alkoxy, Aryloxy oder Halogen bedeuten und
R⁸ und R⁹ unabhängig voneinander Wasserstoff, Alkyl, Alkenyl, Cycloalkyl, Aralkyl, Aryl, einen gegebenenfalls über eine Methylen- oder Ethylenbrücke gebundenen Heterocyclus oder R⁸ und R⁹ gemeinsam mit dem dazwischenliegenden Stickstoffatom einen Heterocyclus bedeuten, wobei
R⁶ und R⁸ und/oder R⁷ und R⁹ gemeinsam auch eine 2- bis 4-gliedrige Brücke bedeuten können, die gegebenenfalls ein Sauerstoff- oder Stickstoffatom enthalten und gegebenenfalls mit 1 bis 3 Alkylgruppen substituiert sein kann und
X^{⊖} ein Anion bedeutet und
wobei alle vorhandenen Alkyl-, Alkenyl-, Cycloalkyl-, Aralkyl-, Aryl-, Alkoxy- und heterocyclischen Reste gegebenenfalls mit nichtionogenen Substituenten, Carboxylgruppen, Ammoniumgruppen und/oder Pyridiniumgruppen substituiert sein können.

2. Kationische 1,3,4-Thiadiazolfarbstoffe nach Anspruch 1, dadurch gekennzeichnet, daß in Formel (I)
R⁵ SO₂R¹⁰ oder COR¹¹ bedeutet, wobei
R¹⁰ für Wasserstoff, Alkyl, Alkenyl, Cycloalkyl, Aralkyl, Aryl oder einen heterocyclischen Rest steht, und
R¹¹ unabhängig von R¹⁰ die gleichen Bedeutungen wie R¹⁰ hat und zusätzlich auch für O-Alkyl, O-Aryl oder NH-R¹⁰ stehen kann.

3. Kationische 1,3,4-Thiadiazolfarbstoffe nach Anspruch 1, dadurch gekennzeichnet, daß in Formel (I)
R¹ und R unabhängig voneinander je einen gegebenenfalls durch Hydroxy, Halogen, Cyano, C₁-C₄-Alkoxy, Aminocarbonyl und/oder C₁-C₄-Alkoxycarbonyl substituierten C₁-C₈-Alkylrest, Allyl, Cyclopentyl, Cyclohexyl, einen gegebenenfalls durch Halogen, Cyano, C₁-C₄-Alkyl und/oder C₁-C₄-Alkoxy substituierten Benzyl- oder Phenethylrest, Thienyl-, Furyl-, Tetrahydrofuryl-, Pyridylmethyl- oder Pyridylethylrest einen gegebenenfalls durch Halogen, Cyano, C₁-C₄-Alkyl und/oder C₁-C₄-Alkoxy substituierten Phenylrest bedeuten, wobei R auch Wasserstoff bedeuten kann, oder
R¹ und R gemeinsam mit dem dazwischenliegenden Stickstoffatom einen gegebenenfalls durch bis zu 4 Methylgruppen substituierten Pyrrolidono-, Piperidino-, Piperazino-oder Morpholinorest bedeuten,
R³ gegebenenfalls durch Hydroxy, Halogen, Cyano, C₁-C₄-Alkoxy, Aminocarbonyl und/oder C₁-C₄-Alkoxycarbonyl substituiertes C₁-C₄-Alkyl, Allyl oder einen gegebenenfalls durch Halogen, C₁-C₄-Alkyl und/oder C₁-C₄-Alkoxy substituierten Benzyl-oder Phenethylrest bedeutet,
R⁴ Wasserstoff oder C₁-C₄-Alkyl bedeutet,
R⁵ SO₂R¹⁰ oder COR¹¹ bedeutet,
R⁶ und R⁷ unabhängig voneinander Wasserstoff, C₁-C₄-Alkyl, C₁-C₄-Alkoxy oder Halogen bedeuten,
R⁸ und R⁹ unabhängig voneinander einen gegebenenfalls durch Hydroxy, Halogen, Cyano, C₁-C₄-Alkoxy, Aminocarbonyl und/oder C₁-C₄-Alkoxycarbonyl substituierten C₁-C₄-Alkylrest, Allyl, Cyclopentyl, Cyclohexyl, einen gegebenenfalls durch Halogen, C₁-C₄-Alkyl, C₁-C₄-Alkoxy, C₁-C₄-Acylamino und/oder Hydroxy substituierten Benzyl-, Phenethyl- oder Phenylrest, Thienyl-, Furyl-, Tetrahydrofuryl-, Pyridyl-, Pyridylmethyl- oder Pyridylethylrest bedeuten, wobei R⁹ auch Wasserstoff bedeuten kann oder
R⁸ und R⁹ gemeinsam mit dem dazwischenliegenden Stickstoffatom auch einen gegebenenfalls durch bis zu 4 Methylgruppen substituierten Pyrrolidino-, Piperidino-, Piperazino-, Morpholino- oder Pyrazolinorest bedeuten kann oder
R⁶ und R⁸ und/oder R⁷ und R⁹ gemeinsam eine solche Brücke bedeuten, daß zusammen mit dem Benzolring, an den R⁶ und/oder R⁷ gebunden sind, und dem Stickstoffatom, an das R⁸ und/oder R⁹ gebunden sind, ein gegebenenfalls durch C₁-C₄-Alkyl substituierter Dihydroindol-, Tetrahydrochinolin-, Tetrahydrochinoxalin-, Tetrahydro-1,4-benzoxazin- oder Julolidinring gebildet wird,
R¹⁰ und R¹¹ unabhängig voneinander je Wasserstoff, gegebenenfalls durch Hydroxy, Halogen, C₁-C₄-Alkoxy und/oder Cyano substituiertes C₁-C₈-Alkyl, C₂-C₄-Alkenyl, Cyclopentyl, Cyclohexyl, einen gegebenenfalls durch Halogen, Cyano, C₁-C₄-Alkyl und/oder C₁-C₄-Alkoxy substituierten Benzyl- oder Phenylrest, Thienyl oder Pyridyl bedeuten, wobei R¹¹ auch O-C₁-bis C₈-Alkyl, einen gegebenenfalls durch Halogen, Cyano, C₁-C₄-Alkyl und/oder C₁-C₄-Alkoxy substituiertes Benzyloxy- oder Phenyloxyrest oder NHR¹⁰ mit R¹⁰ wie hier angegeben bedeuten kann und
X^{⊖} ein Anion bedeutet,

4. Kationische 1,3,4-Thiadiazolfarbstoffe nach Anspruch 1, dadurch gekennzeichnet, daß in Formel (I)
R¹ und R unabhängig voneinander Methyl, Ethyl, Propyl, Butyl, Methylpropyl, Pentyl, Methylbutyl, Dimethylpropyl, Hexyl, Hydroxyethyl, Hydroxypropyl, Chlorethyl, Cyanomethyl, Cyanoethyl, Cyanopropyl, Methoxyethyl, Ethoxyethyl, Methoxypropyl, Aminocarbonylmethyl, Aminocarbonylethyl, Methoxycarbonylmethyl, Methoxycarbonylethyl, Allyl, Cyclohexyl, Benzyl, Phenethyl, Methylbenzyl, Chlorbenzyl, Methoxybenzyl, Phenyl, Tolyl, Chlorphenyl, Anisyl, Cyanophenyl, 2- oder 4-Pyridylmethyl oder 2- oder 4-Pyridylethyl bedeuten und R auch Wasserstoff bedeuten kann oder
R¹ und R gemeinsam mit dem dazwischenliegenden Stickstoffatom Pyrrolidino, Piperidino, Piperazino oder Morpholino bedeuten,
R³ Methyl, Ethyl, Propyl, Butyl, Hydroxyethyl, Hydroxypropyl, Hydroxybutyl, Chlorethyl, Cyanomethyl, Cyanoethyl, Cyanopropyl, Methoxyethyl, Ethoxyethyl, Methoxypropyl, Aminocarbonylethyl, Methoxycarbonylethyl, Ethoxycarbonylethyl, Allyl, Benzyl, Phenethyl, Methylbenzyl, Chlorbenzyl oder Methoxybenzyl bedeutet,
R⁴ Wasserstoff bedeutet,
R⁵ SO₂R¹⁰ oder COR¹¹ bedeutet,
R⁶ Wasserstoff, Methyl, Ethyl, Chlor, Methoxy oder Ethoxy bedeutet,
R⁷ Wasserstoff bedeutet,
R⁸ und R⁹ unabhängig voneinander Methyl, Ethyl, Propyl, Butyl, Hydroxyethyl, Hydroxypropyl, Hydroxybutyl, Chlorethyl, Cyanomethyl, Cyanoethyl, Cyanopropyl, Methoxyethyl, Ethoxyethyl, C₁- bis C₂-Alkylcarbonyloxyethyl, Phenylcarbonyloxyethyl, C₁- bis C₂-Alkyloxycarbonyloxyethyl, Cyan-C₁- bis C₂-alkoxyethyl, Chlorhydroxypropyl, Dihydroxypropyl, Methoxypropyl, Aminocarbonylethyl, Methoxycarbonylethyl, Ethoxycarbonylethyl, Allyl, Benzyl, Phenethyl, Methylbenzyl, Chlorbenzyl, Methoxybenzyl, Phenyl, Methylphenyl, Dimethylphenyl, tert.-Butylphenyl, Chlorphenyl, Dichlorphenyl, Methoxyphenyl, Hydroxymethoxyphenyl, Cyanophenyl, Acetaminophenyl, 2-Thienyl, 2-Tetrahydrofuryl, 2- oder 4-Pyridyl oder 2- oder 4-Pyridylmethyl bedeuten, wobei R⁹ auch Wasserstoff bedeuten kann oder
R⁸ und R⁹ gemeinsam mit dem dazwischenliegenden Stickstoffatom einen unsubstituierten Pyrrolidino-, Piperidino- oder Morpholinorest bedeuten oder
R⁶ und R⁸ gemeinsam eine solche Brücke bedeuten, daß zusammen mit dem Benzolring, an den R⁶ gebunden ist, und dem Stickstoffatom, an das R⁸ gebunden ist, ein Dihydroindol-, Methyldihydroindol-, Tetrahydrochinolin-, Methyltetrahydrochinolin-, Trimethyltetrahydrochinolin- oder Tetrahydro-1,4-benzoxazin-Ring gebildet wird,
R¹⁰ und R¹¹ unabhängig voneinander je Wasserstoff, Methyl, Ethyl, Propyl, Butyl, Hydroxyethyl, Hydroxypropyl, Hydroxybutyl, Cyanoethyl, Methoxyethyl, Vinyl, Allyl, Methylvinyl, Cyclohexyl, Benzyl, Chlorbenzyl, Phenyl, Methylphenyl, Dimethylphenyl, Chlorphenyl, Methoxyphenyl, 2-Thienyl oder 2-, 3- oder 4-Pyridyl bedeuten und
X^{⊖} ein farbloses Anion bedeutet.

5. Kationische 1,3,4-Thiadiazolfarbstoffe nach Anspruch 1, dadurch gekennzeichnet, daß in Formel (I)
R¹ und R unabhängig voneinander Methyl, Ethyl, Propyl, Butyl, Hydroxyethyl, Hydroxypropyl, Cyanoethyl, Methoxyethyl oder Ethoxyethyl bedeuten oder R¹ und R gemeinsam mit dem dazwischenliegenden Stickstoffatom Morpholino bedeuten,
R³ Methyl, Ethyl, Hydroxyethyl, Hydroxypropyl oder Cyanoethyl bedeutet,
R⁴ Wasserstoff bedeutet,
R⁵ COR¹¹ bedeutet,
R⁶ Methyl, Chlor oder Methoxy bedeutet,
R⁷ Wasserstoff bedeutet,
R⁸ Phenyl, Methylphenyl, Dimethylphenyl, tert.-Butylphenyl, Chlorphenyl, Dichlorphenyl, Methoxyphenyl, Hydroxyethoxyphenyl, Cyanophenyl oder Acetaminophenyl bedeutet,
R⁹ Wasserstoff bedeutet,
R¹¹ Methyl oder Ethyl bedeutet und
X^{⊖} ein farbloses Anion bedeutet.

6. Anhydrobasen der Formeln (II) und (III) 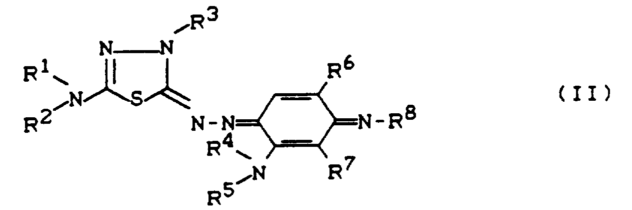 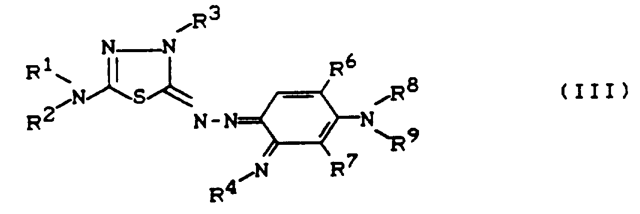 in denen R¹ bis R⁹ die in Anspruch 1 angebene Bedeutung haben.

7. Verfahren zur Herstellung von kationischen 1,3,4-Thiadiazolfarbstoffen des Anspruchs 1, dadurch gekennzeichnet, daß man 2-Amino-1,3,4-thiadiazole der Formel (IV) 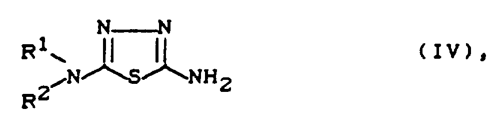 in der
R¹ und R die im Anspruch 1 angegebene Bedeutung haben,
auf m-Phenylendiaminderivate der Formel (V) 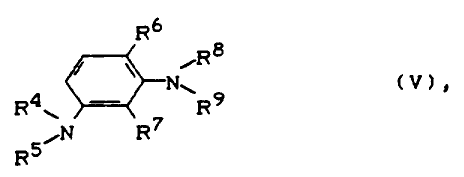 in der
R⁴ bis R⁹ die im Anspruch 1 angegebene Bedeutung haben,
kuppelt und dann mit Verbindungen der Formel (VI)
(R³)X (VI),
in der
R³ und X die in Anspruch 1 angegebene Bedeutung haben,
quarterniert.

8. Verfahren zur Herstellung von kationischen 1,3,4-Thiadiazolfarbstoffen des Anspruchs 1, dadurch gekennzeichnet, daß man Farbstoffe der Formel (VII) 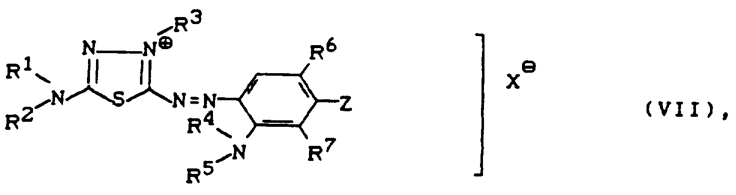 in der
R¹ bis R⁷ und X^{⊖} die im Anspruch 1 angegebene Bedeutung haben und
Z für Halogen, Hydroxy, Alkoxy, Cycloalkoxy, Aryloxy, Amino oder Dialkylamino steht,
mit Aminen der Formel (VIII) 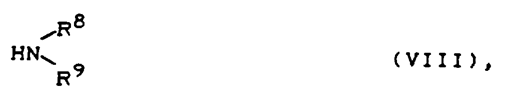 in der
R⁸ und R⁹ die in Anspruch 1 angegebene Bedeutung haben,
umsetzt.

9. Verwendung von kationischen 1,3,4-Thiadiazolfarbstoffen des Anspruchs 1 zum Färben und Bedrucken von kationisch färbbaren Fasern, tannierten Zellulosematerialien, Papier, Seide und Leder, sowie zur Herstellung von Kugelschreiberpasten, Schreib- und Stempelflüssigkeiten und im Gummidruck.

10. Kationisch färbbare Fasern, tannierte Zellulosematerialien, Papier, Seide, Leder, Kugelschreiberpasten, Schreibflüssigkeiten und Stempelflüssigkeiten, dadurch gekennzeichnet, daß sie mindestens einen kationischen 1,3,4-Thiadiazolfarbstoff des Anspruchs 1 enthalten.

## Claims

1. Cationic 1,3,4-thiadiazole dyestuffs of the formula (I) 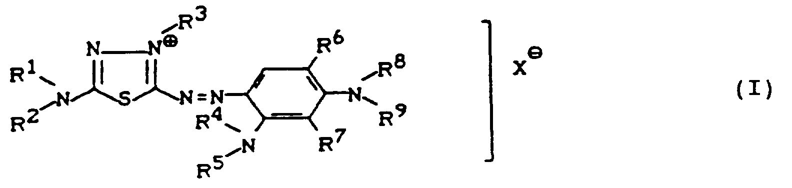 in which
R¹ and R, independently of one another, denote hydrogen, alkyl, alkenyl, cycloalkyl, aralkyl, aryl, a heterocycle which may be bound via a methylene or ethylene bridge or R¹ and R, together with the nitrogen atom in between denote a heterocycle,
R³ denotes alkyl, alkenyl or aralkyl,
R⁴ denotes hydrogen or alkyl,
R⁵ denotes an acyl group,
R⁶ and R⁷, independently of one another, denote hydrogen, alkyl, alkoxy, aryloxy or halogen and
R⁸ and R⁹, independently of one another, denote hydrogen, alkyl, alkenyl, cycloalkyl, aralkyl, aryl, a heterocycle which may bound via a methylene or ethylene bridge or R⁸ and R⁹, together with the nitrogen atom in between, denote a heterocycle, in which
R⁶ and R⁸ and/or R⁷ and R⁹ together can also denote a 2- to 4-membered bridge which may contain an oxygen or nitrogen atom and may be substituted by 1 to 3 alkyl groups and
X⊖ denotes an anion and
in which all alkyl, alkenyl, cycloalkyl, aralkyl, aryl, alkoxy and heterocyclic radicals present may be substituted by nonionic substituents, carboxyl groups, ammonium groups and/or pyridinium groups.

2. Cationic 1,3,4-thiadiazole dyestuffs according to Claim 1, characterised in that in formula (I)
R⁵ denotes SO₂R¹⁰ or COR¹¹ in which
R¹⁰ represents hydrogen, alkyl, alkenyl, cycloalkyl, aralkyl, aryl or a heterocyclic radical, and
R¹¹, independently of R¹⁰, has the same meanings as R¹⁰ and can additionally also represent O-alkyl, O-aryl or NH-R¹⁰.

3. Cationic 1,3,4-thiadiazole dyestuffs according to Claim 1, characterised in that in formula (I)
R¹ and R, independently of one another, each denote a C₁-C₈-alkyl radical which is unsubstituted or substituted by hydroxyl, halogen, cyano, C₁-C₄-alkoxy, aminocarbonyl and/or C₁-C₄-alkoxycarbonyl, or denote allyl, cyclopentyl, cyclohexyl, an unsubstituted or halogen-, cyano-, C₁-C₄-alkyl- and/or C₁-C₄-alkoxy-substituted benzyl or phenethyl radical, thienyl, furyl, tetrahydrofuryl, pyridylmethyl or pyridylethyl radical, an unsubstituted or halogen-, cyano-, C₁-C₄-alkyl- and/or C₁-C₄-alkoxy-substituted phenyl radical, in which R can also denote hydrogen, or
R¹ and R, together with the nitrogen atom in between, denote a pyrrolidono, piperidino, piperazino or morpholino radical which may be substituted by up to 4 methyl groups,
R³ denotes unsubstituted or hydroxyl-, halogen-, cyano-, C₁-C₄-alkoxy-, aminocarbonyl- and/or C₁-C₄-alkoxycarbonylsubstituted C₁-C₄-alkyl, allyl or an unsubstituted or halogen-, C₁-C₄-alkyl-and/or C₁-C₄-alkoxy-substituted benzyl or phenethyl radical,
R⁴ denotes hydrogen or C₁-C₄-alkyl,
R⁵ denotes SO₂R¹⁰ or COR¹¹,
R⁶ and R⁷, independently of one another, are, hydrogen C₁-C₄-alkyl, C₁-C₄-alkoxy or halogen,
R⁸ and R⁹, independently of one another, denote an unsubstituted or hydroxyl-, halogen-, cyano-, C₁-C₄-alkoxy-, aminocarbonyl-and/or C₁-C₄-alkoxycarbonyl-substituted C₁-C₄-alkyl radical, allyl, cyclopentyl, cyclohexyl, an unsubstituted or halogen-, C₁-C₄-alkyl-, C₁-C₄-alkoxy-, C₁-C₄-acylamino- and/or hydroxyl-substituted benzyl, phenethyl or phenyl radical, thienyl, furyl, tetrahydrofuryl, pyridyl, pyridylmethyl or pyridylethyl radical, in which R⁹ can also denote hydrogen or
R⁸ and R⁹, together with the nitrogen atom in between, can also denote a pyrrolidino, piperidino, piperazino, morpholino or pyrazolino radical which is unsubstituted or substituted by up to 4 methyl groups, or
R⁶ and R⁸ and/or R⁷ and R⁹ together denote such a bridge that together with the benzene ring to which R⁶ and/or R⁷ are bound and the nitrogen atom to which R⁸ and/or R⁹ are bound an unsubstituted or C₁-C₄-alkyl-substituted dihydroindole, tetrahydroquinoline, tetrahydroquinoxaline, tetrahydro-1,4-benzoxazine or julolidine ring is formed,
R¹⁰ and R¹¹, independently of one another, each denote hydrogen, unsubstituted or hydroxy-, halogen-, C₁-C₄-alkoxy- and/or cyano-substituted C₁-C₈-alkyl, C₂-C₄-alkenyl, cyclopentyl, cyclohexyl, an unsubstituted or halogen-, cyano-, C₁-C₄-alkyl- and/or C₁-C₄-alkoxy-substituted benzyl or phenyl radical, thienyl or pyridyl, in which R¹¹ can also denote O-C₁- to C₈-alkyl, an unsubstituted or halogen-, cyano-, C₁-C₄-alkyl- and/or C₁-C₄-alkoxy-substituted benzyloxy or phenyloxy radical or NHR¹⁰ where R¹⁰ can have the meaning given here and
X^{⊖} denotes an anion.

4. Cationic 1,3,4-thiadiazole dyestuffs according to Claim 1, characterised in that in formula (I),
R¹ and R, independently of one another, denote methyl, ethyl, propyl, butyl, methylpropyl, pentyl, methylbutyl, dimethylpropyl, hexyl, hydroxyethyl, hydroxypropyl, chloroethyl, cyanomethyl, cyanoethyl, cyanopropyl, methoxyethyl, ethoxyethyl, methoxypropyl, aminocarbonylmethyl, aminocarbonylethyl, methoxycarbonylmethyl, methoxycarbonylethyl, allyl, cyclohexyl, benzyl, phenethyl, methylbenzyl, chlorobenzyl, methoxybenzyl, phenyl, tolyl, chlorophenyl, anisyl, cyanophenyl, 2- or 4-pyridylmethyl or 2- or 4-pyridylethyl and R can also denote hydrogen or
R¹ and R, together with the nitrogen atom in between, denote pyrrolidino, piperidino, piperazino or morpholino,
R³ denotes methyl, ethyl, propyl, butyl, hydroxyethyl, hydroxypropyl, hydroxybutyl, chloroethyl, cyanomethyl, cyanoethyl, cyanopropyl, methoxyethyl, ethoxyethyl, methoxypropyl, aminocarbonylethyl, methoxycarbonylethyl, ethoxycarbonylethyl, allyl, benzyl, phenethyl, methylbenzyl, chlorobenzyl or methoxybenzyl,
R⁴ denotes hydrogen,
R⁵ denotes SO₂R¹⁰ or COR¹¹,
R⁶ denotes hydrogen, methyl, ethyl, chlorine, methoxy or ethoxy,
R⁷ denotes hydrogen,
R⁸ and R⁹, independently of one another, denote methyl, ethyl, propyl, butyl, hydroxyethyl, hydroxypropyl, hydroxybutyl, chloroethyl, cyanomethyl, cyanoethyl, cyanopropyl, methoxyethyl, ethoxyethyl, C₁- to C₂-alkylcarbonyloxyethyl, phenylcarbonyloxyethyl, C₁- to C₂-alkoxycarbonyloxyethyl, cyano-C₁- to C₂-alkoxyethyl, chloro-hydroxypropyl, dihydroxypropyl, methoxypropyl, aminocarbonylethyl, methoxycarbonylethyl, ethoxycarbonylethyl, allyl, benzyl, phenethyl, methylbenzyl, chlorobenzyl, methoxybenzyl, phenyl, methylphenyl, dimethylphenyl, tert.-butylphenyl, chlorophenyl, dichlorophenyl, methoxyphenyl, hydroxymethoxyphenyl, cyanophenyl, acetaminophenyl, 2-thienyl, 2-tetrahydrofuryl, 2-or 4-pyridyl or 2- or 4-pyridylmethyl, in which R⁹ can also denote hydrogen, or
R⁸ and R⁹, together with the nitrogen atom in between denote an unsubstituted pyrrolidino, piperidino or morpholino radical or
R⁶ and R⁸ together denote such a bridge that together with the benzene ring to which R⁶ is bound and the nitrogen atom to which R⁸ is bound a dihydroindole, methyldihydroindole, tetrahydroquinoline, methyltetrahydroquinoline, trimethyltetrahydroquinoline or tetrahydro-1,4-benzoxazine ring is formed,
R¹⁰ and R¹¹, independently of one another, are each hydrogen, methyl, ethyl, propyl, butyl, hydroxyethyl, hydroxypropyl, hydroxybutyl, cyanoethyl, methoxyethyl, vinyl, allyl, methylvinyl, cyclohexyl, benzyl, chlorobenzyl, phenyl, methylphenyl, dimethylphenyl, chlorophenyl, methoxyphenyl, 2-thienyl or 2-, 3- or 4-pyridyl and
X^{⊖} denotes a colourless anion.

5. Cationic 1,3,4-thiadiazole dyestuffs according to Claim 1, characterised in that in formula (I)
R¹ and R, independently of one another, denote methyl, ethyl, propyl, butyl, hydroxyethyl, hydroxypropyl, cyanoethyl, methoxyethyl or ethoxyethyl or R¹ and R, together with the nitrogen atom in between, denote morpholino,
R³ denotes methyl, ethyl, hydroxyethyl, hydroxypropyl or cyanoethyl,
R⁴ denotes hydrogen,
R⁵ denotes COR¹¹,
R⁶ denotes methyl, chlorine or methoxy,
R⁷ denotes hydrogen,
R⁸ denotes phenyl, methylphenyl, dimethylphenyl, tert.-butylphenyl, chlorophenyl, dichlorophenyl, methoxyphenyl, hydroxyethoxyphenyl, cyanophenyl or acetaminophenyl,
R⁹ denotes hydrogen,
R¹¹ denotes methyl or ethyl and
X^{⊖} denotes a colourless anion.

6. Anhydro bases of the formulae (II) and (III) 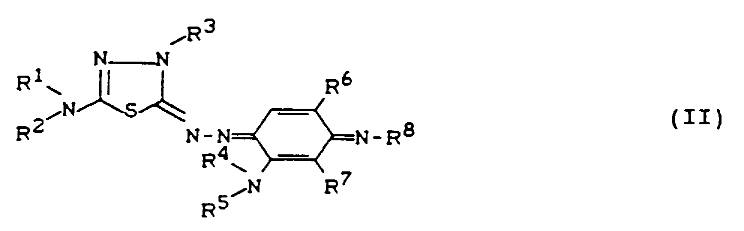 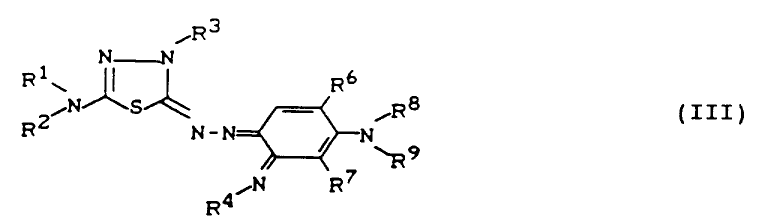 in which R¹ to R⁹ have the meaning given in Claim 1.

7. Process for the preparation of cationic 1,3,4-thiadiazole dyestuffs of Claim 1, characterised in that 2-amino-1,3,4-thiadiazoles of the formula (IV) 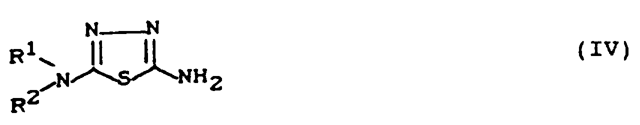 in which
R¹ and R have the meaning given in Claim 1, are coupled onto m-phenylenediamine derivatives of the formula (V) 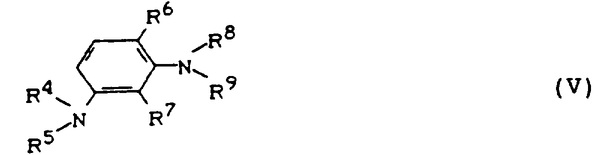 in which
R⁴ to R⁹ have the meaning given in Claim 1, and the products are then quaternised with compounds of the formula (VI)
(R³)X (VI)
in which
R³ and X have the meaning given in Claim 1.

8. Process for the preparation of cationic 1,3,4-thiadiazole dyestuffs of Claim 1, characterised in that dyestuffs of the formula (VII) 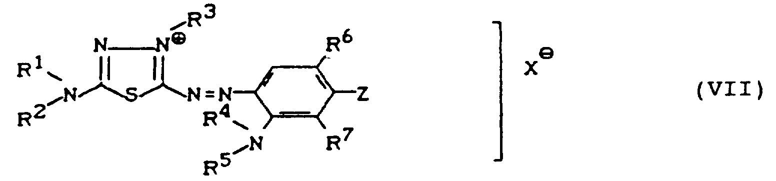 in which
R¹ to R⁷ and X^{⊖} have the meaning given in Claim 1 and
Z represents halogen, hydroxyl, alkoxy, cycloalkoxy, aryloxy, amino or dialkylamino,
are reacted with amines of the formula (VIII) 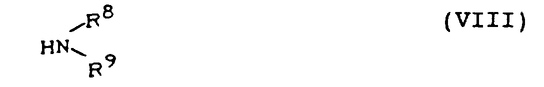 in which
R⁸ and R⁹ have the meaning given in Claim 1.

9. Use of cationic 1,3,4-thiadiazole dyestuffs of Claim 1 for the dyeing and printing of cationic dyeable fibres, cellulose materials treated with tannic acid, paper, silk and leather, and for the preparation of ball point pen pastes, writing and stamping inks and in flexographic printing.

10. Cationically dyeable fibres, cellulose materials treated with tannic acid, paper, silk, leather, ball point pen pastes, writing inks and stamping inks, characterised in that they contain at least one cationic 1,3,4-thiadiazole dyestuff of Claim 1.

## Revendications

1. Colorants cationiques 1,3,4-thiadiazoliques de formule (I) 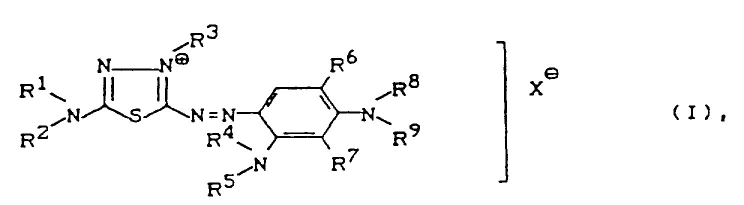 dans laquelle
R¹ et R représentent chacun, indépendamment l'un de l'autre, l'hydrogène, un groupe alkyle, alcényle, cycloalkyle, aralkyle, aryle, un hétérocycle éventuellement relié par l'intermédiaire d'un pont méthylène ou éthylène, ou bien R¹ et R forment ensemble et avec l'atome d'azote auquel ils sont reliés un hétérocycle,
R³ représente un groupe alkyle, alcényle ou aralkyle,
R⁴ représente l'hydrogène ou un groupe alkyle,
R⁵ représente un groupe acyle,
R⁶ et R⁷ représentent chacun, indépendamment l'un de l'autre, l'hydrogène, un groupe alkyle, alcoxy, aryloxy ou un halogène et
R⁸ et R⁹ représentent chacun, indépendamment l'un de l'autre, l'hydrogène, un groupe alkyle, alcényle, cycloalkyle, aralkyle, aryle, un hétérocycle éventuellement relié par l'intermédiaire d'un pont méthylène ou éthylène, ou bien R⁸ et R⁹ forment ensemble et avec l'atome d'azote auquel ils sont reliés un hétérocycle,
R⁶ et R⁸ et/ou R⁷ et R⁹ pouvant également former ensemble un pont de 2 à 4 chaînons qui peut contenir le cas échéant un atome d'oxygène ou d'azote et qui peut le cas échéant être substitué par 1 à 3 groupes alkyles et
X^{⊖} représente un anion,
tous les radicaux alkyle, alcényle, cycloalkyle, aralkyle, aryle, alcoxy et hétérocycles présents pouvant porter le cas échéant des substituants non ioniques, des groupes carboxyles, des groupes ammonium et/ou des groupes pyridiniums.

2. Colorants cationiques 1,3,4-thiadiazoliques selon la revendication 1, caractérisés en ce que, dans la formule (I)
R⁵ représente un groupe SO₂R¹⁰ ou COR¹¹ dans lesquels
R¹⁰ représente l'hydrogène, un groupe alkyle, alcényle, cycloalkyle, aralkyle, aryle ou un radical hétérocyclique et
R¹¹ a, indépendamment de R¹⁰, les mêmes significations que celui-ci mais peut en outre représenter un groupe O-alkyle, O-aryle ou NH-R¹⁰.

3. Colorants cationiques 1,3,4-thiadiazoliques selon la revendication 1, caractérisés en ce que, dans la formule (I)
R¹ et R représentent chacun, indépendamment l'un de l'autre, un groupe alkyle en C₁-C₈ éventuellement substitué par des groupes hydroxy, des halogènes, des groupes cyano, alcoxy en C₁-C₄, aminocarbonyle et/ou (alcoxy en C₁₋C₄)carbonyle, un groupe allyle, cyclopentyle, cyclohexyle, un groupe benzyle ou phénéthyle éventuellement substitué par des halogènes, des groupes cyano, alkyle en C₁-C₄ et/ou alcoxy en C₁-C₄, un groupe thiényle, furyle, tétrahydrofuryle, pyridylméthyle ou pyridyléthyle, un groupe phényle éventuellement substitué par des halogènes, des groupes cyano, alkyle en C₁-C₄ et/ou alcoxy en C₁-C₄, R pouvant également représenter l'hydrogène, ou bien
R¹ et R forment ensemble et avec l'atome d'azote auquel ils sont reliés un groupe pyrrolidino, pipéridino, pipérazino ou morpholino portant éventuellement jusqu'à 4 substituants méthyle,
R³ représente un groupe alkyle en C₁-C₄ éventuellement substitué par des groupes hydroxy, des halogènes, des groupes cyano, alcoxy en C₁-C₄, aminocarbonyle et/ou (alcoxy en C₁-C₄)carbonyle, un groupe allyle, ou un groupe benzyle ou phénéthyle éventuellement substitué par des halogènes, des groupes alkyles en C₁-C₄ et/ou alcoxy en C₁-C₄,
R⁴ représente l'hydrogène ou un groupe alkyle en C₁ -C₄,
R⁵ représente SO₂R¹⁰ ou COR¹¹,
R⁶ et R⁷ représentent chacun, indépendamment l'un de l'autre, l'hydrogène, un groupe alkyle en C₁ -C₄, alcoxy en C₁-C₄ ou un halogène,
R⁸ et R⁹ représentent chacun, indépendamment l'un de l'autre, un groupe alkyle en C₁-C₄ éventuellement substitué par des groupes hydroxy, des halogènes, des groupes cyano, alcoxy en C₁-C₄, aminocarbonyle et/ou (alcoxy en C₁-C₄) carbonyle, un groupe allyle, cyclopentyle, cyclohexyle, un groupe benzyle, phénéthyle ou phényle éventuellement substitué par des halogènes, des groupes alkyle en C₁-C₄, alcoxy en C₁-C₄, acylamino en C₁-C₄ et/ou hydroxy, un groupe thiényle, furyle, tétrahydrofuryle, pyridyle, pyridylméthyle ou pyridyléthyle, R⁹ pouvant également représenter l'hydrogène, ou bien
R⁸ et R⁹ peuvent former ensemble et avec l'atome d'azote auquel ils sont reliés un groupe pyrrolidino, pipéridino, pipérazino, morpholino ou pyrazolino portant éventuellement jusqu'à 4 substituants méthyle, ou bien
R⁶ et R⁸ et/ou R⁷ et R⁹ peuvent former ensemble un pont qui forme lui même, avec le cycle benzénique portant R⁶ et/ou R⁷, et l'atome d'azote auquel R⁸ et/ou R⁹ sont reliés, un cycle dihydroindole, tétrahydroquinoléine, tétrahydroquinoxaline, tétrahydro-1,4-benzoxazine ou julolidine éventuellement substitué par un groupe alkyle en C₁-C₄,
R¹⁰ et R¹¹ représentent chacun, indépendamment l'un de l'autre, l'hydrogène, un groupe alkyle en C₁-C₈ éventuellement substitué par des groupes hydroxyle, des halogènes, des groupes alcoxy en C₁-C₄ et/ou cyano, un groupe alcényle en C₂-C₄, cyclopentyle, cyclohexyle, un groupe benzyle ou phényle éventuellement substitué par des halogènes, des groupes cyano, alkyle en C₁-C₄ et/ou alcoxy en C₁-C₄, un groupe thiényle ou pyridyle, R¹¹ pouvant également représenter un groupe O-alkyle en C₁-C₈, un groupe benzyloxy ou phényloxy éventuellement substitué par des halogènes, des groupes cyano, alkyle en C₁-C₄ et/ou alcoxy en C₁-C₄, ou bien NHR¹⁰ dans lequel R¹⁰ a les significations indiquées ci-dessus et
X^{⊖} représente un anion.

4. Colorants cationiques 1,3,4-thiadiazoliques selon la revendication 1, caractérisés en ce que, dans la formule (I)
R¹ et R représentent chacun, indépendamment l'un de l'autre, un groupe méthyle, éthyle, propyle, butyle, méthylpropyle, pentyle, méthylbutyle, diméthylpropyle, hexyle, hydroxyéthyle, hydroxypropyle, chloréthyle, cyanométhyle, cyanoéthyle, cyanopropyle, méthoxyéthyle, éthoxyéthyle, méthoxypropyle, aminocarbonylméthyle, aminocarbonyléthyle, méthoxycarbonylméthyle, méthoxycarbonyléthyle, allyle, cyclohexyle, benzyle, phénéthyle, méthylbenzyle, chlorobenzyle, méthoxybenzyle, phényle, tolyle, chlorophényle, anisyle, cyanophényle, 2- ou 4-pyridylméthyle ou 2- ou 4-pyridyléthyle, R pouvant également représenter l'hydrogène, ou bien
R¹ et R forment ensemble et avec l'atome d'azote auquel ils sont reliés un groupe pyrrolidino, pipéridino, pipérazino ou morpholino,
R³ représente un groupe méthyle, éthyle, propyle, butyle, hydroxyéthyle, hydroxypropyle, hydroxybutyle, chloréthyle, cyanométhyle, cyanoéthyle, cyanopropyle, méthoxyéthyle, éthoxyéthyle, méthoxypropyle, aminocarbonyléthyle, méthoxycarbonyléthyle, éthoxycarbonyléthyle, allyle, benzyle, phénéthyle, méthylbenzyle, chlorobenzyle ou méthoxybenzyle,
R⁴ représente l'hydrogène,
R⁵ représente SO₂R¹⁰ ou COR¹¹,
R⁶ représente l'hydrogène, un groupe méthyle, éthyle, le chlore, un groupe méthoxy ou éthoxy,
R⁷ représente l'hydrogène,
R⁸ et R⁹ représentent chacun, indépendamment l'un de l'autre, un groupe méthyle, éthyle, propyle, butyle, hydroxyéthyle, hydroxypropyle, hydroxybutyle, chloréthyle, cyanométhyle, cyanoéthyle, cyanopropyle, méthoxyéthyle, éthoxyéthyle, (alkyle en C₁-C₂)carbonyloxyéthyle, phénylcarbonyloxyéthyle, (alkyle en C₁-C₂)oxycarbonyloxyéthyle, cyano-(alcoxy en C₁-C₂)éthyle, chlorhydroxypropyle, dihydroxypropyle, méthoxypropyle, aminocarbonyléthyle, méthoxycarbonyléthyle, éthoxycarbonyléthyle, allyle, benzyle, phénéthyle, méthylbenzyle, chlorobenzyle, méthoxybenzyle, phényle, méthylphényle, diméthylphényle, tert-butylphényle, chlorophényle, dichlorophényle, méthoxyphényle, hydroxyméthoxyphényle, cyanophényle, acétaminophényle, 2-thiényle, 2-tétrahydrofuryle, 2- ou 4-pyridyle ou 2- ou 4-pyridylméthyle, R⁹ pouvant également représenter l'hydrogène, ou bien
R⁸ et R⁹ forment ensemble et avec l'atome d'azote auquel ils sont reliés un groupe pyrrolidino, pipéridino ou morpholino non substitué, ou bien
R⁶ et R⁸ forment ensemble un pont qui, avec le cycle benzénique portant R⁶ et l'atome d'azote auquel R⁸ est relié, forme lui même un cycle dihydroindole, méthyldihydroindole, tétrahydroquinoléine, méthyltétrahydroquinoléine, triméthyltétrahydroquinoléine ou tétrahydro-1,4-benzoxazine, et
R¹⁰ et R¹¹ représentent chacun, indépendamment l'un de l'autre, l'hydrogène, un groupe méthyle, éthyle, propyle, butyle, hydroxyéthyle, hydroxypropyle, hydroxybutyle, cyanoéthyle, méthoxyéthyle, vinyle, allyle, méthylvinyle, cyclohexyle, benzyle, chlorobenzyle, phényle, méthylphényle, diméthylphényle, chlorophényle, méthoxyphényle, 2-thiényle ou 2-, 3- ou 4-pyridyle et
X^{⊖} représente un anion incolore.

5. Colorants cationiques 1,3,4-thiadiazoliques selon la revendication 1, caractérisé en ce que, dans la formule (I)
R¹ et R représentent chacun, indépendamment l'un de l'autre, un groupe méthyle, éthyle, propyle, butyle, hydroxyéthyle, hydroxypropyle, cyanoéthyle, méthoxyéthyle ou éthoxyéthyle, ou bien R¹ et R forment ensemble avec l'atome d'azote auquel ils sont reliés un cycle morpholino,
R³ représente un groupe méthyle, éthyle, hydroxyéthyle, hydroxypropyle ou cyanoéthyle,
R⁴ représente l'hydrogène,
R⁵ représente COR¹¹
R⁶ représente un groupe méthyle, le chlore ou un groupe méthoxy,
R⁷ représente l'hydrogène,
R⁸ représente un groupe phényle, méthylphényle, diméthylphényle, tertbutylphényle, chlorophényle, dichlorophényle, méthoxyphényle, hydroxyéthoxyphényle, cyanophényle ou acétaminophényle,
R⁹ représente l'hydrogène,
R¹¹ représente un groupe méthyle et éthyle et
X^{⊖} représente un anion incolore.

6. Anhydrobases de formules (II) et (III) 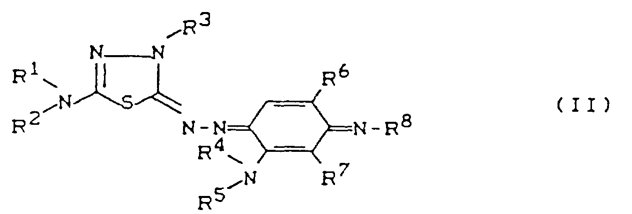 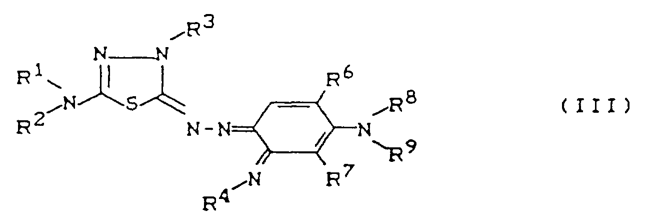 dans lesquelles R¹ à R⁹ ont les significations indiquées dans la revendication 1.

7. Procédé de préparation des colorants cationiques 1,3,4-thiadiazolique de la revendication 1, caractérisé en ce que l'on condense des 2-amino-1,3,4-thidiazoles de formule (IV) 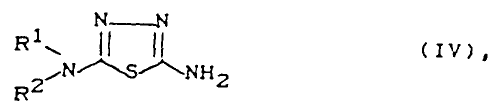 dans laquelle
R¹ et R ont les significations indiquées dans la revendication 1, avec des dérivés de la m-phénylènediamine de formule (V) 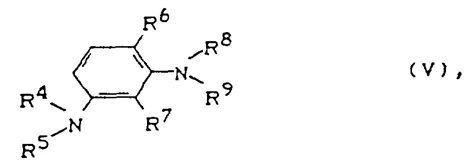 dans laquelle
R⁴ à R⁹ ont les significations indiquées dans la revendication 1, puis on quatemise à l'aide de composés de formule (VI)
(R³)X (VI),
dans laquelle
R³ et X ont les significations indiquées dans la revendication 1.

8. Procédé de préparation des colorants cationiques 1,3,4-thiadiazoliques de la revendication 1, caractérisé en ce que l'on fait réagir les colorants de formule (VII) 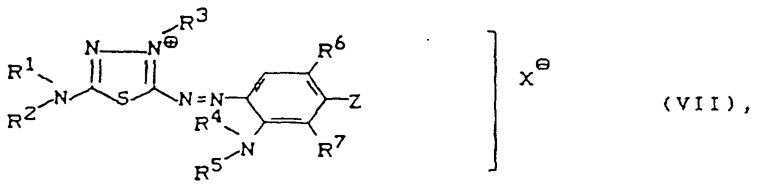 dans laquelle
R¹ à R⁷ et X^{⊖} ont les significations indiquées dans la revendication 1 et
Z représente un halogène, un groupe hydroxy, alcoxy, cycloalcoxy, aryloxy, amino ou dialkylamino,
avec des amines de formule (VIII) 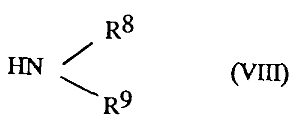 dans laquelle
R⁸ et R⁹ ont les significations indiquées dans la revendication 1.

9. Utilisation des colorants cationiques 1,3,4-thiadiazoliques de la revendication 1 pour la teinture et l'impression de fibres aptes à la teinture par des colorants cationiques, de matières cellulosiques tannées, du papier, de la soie et du cuir, et pour la préparation de pâte pour stylos à billes, d'encres de toutes natures et d'encres pour tampons et dans l'impression par caoutchouc.

10. Fibres aptes à la teinture par des colorants cationiques, matières cellulosiques tannées, papier, soie, cuir, pâtes pour stylos à billes, encres de toutes natures et encres pour tampons, caractérisés en ce qu'ils contiennent au moins un colorant cationique 1,3,4-thiadiazolique de la revendication 1.
